# EUROPEAN PATENT APPLICATION

(11) **EP 1 997 514 A1**
(43) Date of publication of application: **03.12.2008**
(21) Application number: 08015462.8
(22) Date of filing: 26.03.1998
(51) Int. Cl.: A61K 47/48, C07K 16/00, C07K 16/10, C12N 15/13

(54) **High avidity polyvalent and polyspecific reagents**

(30) Priority: 27.03.1997 AU PO591797
(62) Divisional of application: 98910534.1
(71) Applicant: AVIPEP PTY LIMITED, South Melbourne, VIC 3205 (AU)
(72) Inventor: Hudson, Peter John, Blackburn Victoria 3130 (AU); Kortt, Alex Andrew, Strathmore Victoria 3041 (AU); Irving, Robert Alexander, Mulgrave Victoria 3170 (AU); Atwell, John Leslie, Vermont South Victoria 3133 (AU)
(74) Representative: Bramley, Sarah Jane

(57) **Abstract**

This application provides polyvalent or polyspecific protein complexes, comprising three or more polypeptides which associate to form three or more functional target-binding regions (TBRs), and in which each individual polypeptide comprises two or more immunoglobulin-like domains which are covalently joined together, such that two Ig-like domains in a single polypeptide do not associate with each other to form a TBR. By using a linker peptide of fewer than three amino acid residues the immunoglobulin-like domains of the individual polypeptides are prevented from associating, so that complex formation between polypeptides is favoured. Preferably the polyvalent or polyspecific protein is a trimer or tetramer. The proteins of the application have specifities which may be the same or different, and are suitable for use as therapeutic, diagnostic or imaging agents.

## Description

This invention relates to target-binding polypeptides, especially polypeptides of high avidity and multiple specificity. In particular the invention relates to protein complexes which are polyvalent and/or polyspecific, and in which the specificity is preferably provided by the use of immunoglobulin-like domains. In one particularly preferred embodiment the protein complex is trivalent and/or trispecific.

### BACKGROUND OF THE INVENTION

Reagents having the ability to bind specifically to a predetermined chemical entity are widely used as diagnostic agents or for targeting of chemotherapeutic agents. Because of their exquisite specificity, antibodies, especially monoclonal antibodies, have been very widely used as the source of the chemical binding specificity.

Monoclonal antibodies are derived from an isolated cell line such as hybridoma cells; however, the hybridoma technology is expensive, time-consuming to maintain and limited in scope. It is not possible to produce monoclonal antibodies, much less monoclonal antibodies of the appropriate affinity, to a complete range of target antigens.

Antibody genes or fragments thereof can be cloned and expressed in *E*. *coli* in a biologically functional form. Antibodies and antibody fragments can also be produced by recombinant DNA technology using either bacterial or mammalian cells. The hapten- or antigen-binding site of an antibody, referred to herein as the target-binding region (TBR), is composed of amino acid residues provided by up to six variable surface loops at the extremity of the molecule.

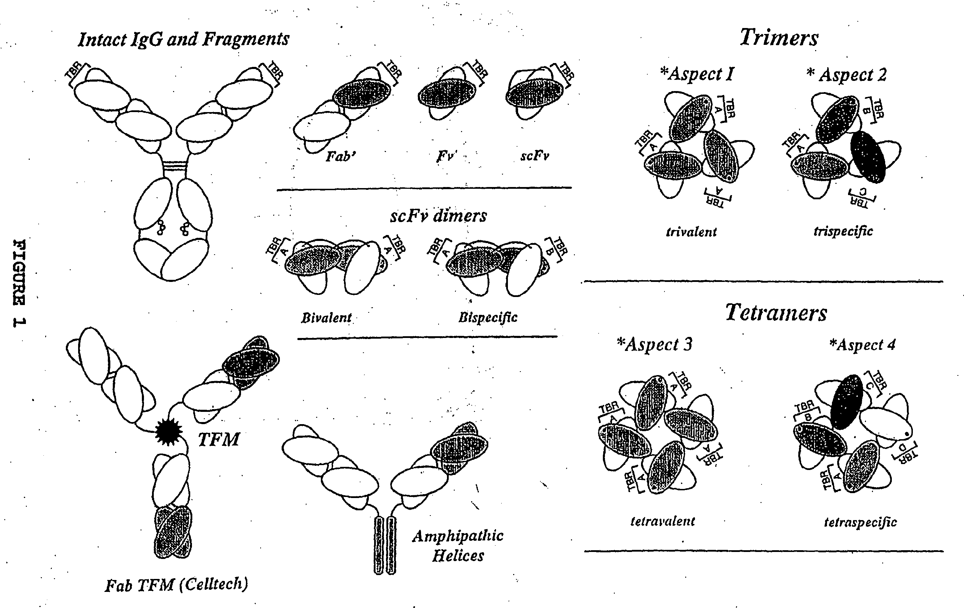

These loops in the outer domain (Fv) are termed complementarity-determining regions (CDRs), and provide the specificity of binding of the antibody to its antigenic target. This binding function is localised to the variable domains of the antibody molecule, which are located at the amino-terminal end of both the heavy and light chains.
This is illustrated in Figure 1. The variable regions of some antibodies remain;non-covalently associated (as V_{H}V_{L} dimers, termed Fv regions) even after proteolytic cleavage from the native antibody molecule, and retain much of their antigen recognition and binding capabilities. Methods of manufacture of Fv region substantially free of constant region are disclosed in US-4,642,334.

Recombinant Fv fragments are prone to dissociation, and therefore some workers have chosen to covalently link the two domains to form a construct designated scFv, in which two peptides with binding domains (usually antibody heavy and light variable regions) are joined by a linker peptide connecting the C-terminus of one domain to the N-terminus of the other, so that the relative positions of the antigen binding domains are consistent with those found in the original antibody (see Figure 1).

Methods of manufacture of covalently linked Fv fragments are disclosed in US-4,946,778 and US-5,132,405. Further heterogeneity can be achieved by the production of bifunctional and multifunctional agents (Huston et al U.S. Patent No. 5,091,513, and Ladner et al U.S. Patent No. 4,816,397).

The construction of scFv libraries is disclosed for example in European Patent Application No. 239400 and U.S. Patent No.4,946,778. However, single-chain Fv libraries are limited in size because of problems inherent in the cloning of a single DNA molecule encoding the scFv. Non-scFv libraries, such as V_{H} or Fab libraries, are also known (Ladner and Guterman WO 90/02809), and may be used with a phage system for surface expression (Ladner *et al* WO 88/06630 and Bonnert et *al* WO 92/01047).

For use in antibody therapy, monoclonal antibodies, which are usually of mouse origin, have limited use unless they are first "humanised", because they elicit an antigenic response on administration to humans. The variable domains of an antibody consist of a β-sheet framework with six hypervariable regions (CDRs) which fashion the antigen-binding site. Humanisation consists of substituting mouse sequences that provide the binding affinity, particularly the CDR loop sequences, into a human variable domain structure. The murine CDR loop regions can therefore provide the binding affinities for the required antigen. Recombinant antibody "humanisation" by grafting of CDRs is disclosed by Winter et al (EP-239400).

The expression of diverse recombinant human antibodies by the use of expression/combinatorial systems has been described (Marks *et al*, 1991). Recent developments in methods for the expression of peptides and proteins on the surface of filamentous phage (McCafferty *et al*, 1991; Clackson et *al,* 1991) offer the potential for the selection, improvement and development of these reagents as diagnostics and therapeutics. The use of modified bacteriophage genomes for the expression, presentation and pairing of cloned heavy and light chain genes of both mouse and human origins has been described (Hoogenboom *et al*, 1991; Marks *et al*,1991 *op.cit*. and Bonnert *et al*, WPI Acc. No. 92-056862/07)

Receptor molecules, whose expression is the result of the receptor-coding gene library in the expressing organism, may also be displayed in the same way (Lerner and Sorge, WO 90/14430). The cell surface expression of single chain antibody domains fused to a cell surface protein is disclosed by Ladner et al, WO 88/06630.

Affinity maturation is a process whereby the binding specificity, affinity or avidity of an antibody can be modified. A number of laboratory techniques have been devised whereby amino acid sequence diversity is created by the application of various mutation strategies, either on the entire antibody fragment or on selected regions such as the CDRs. Mutation to change enzyme specific activity has also been reported. The person skilled in the art will be aware of a variety of methods for achieving random or site-directed mutagenesis, and for selecting molecules with a desired modification. Mechanisms to increase diversity and to select specific antibodies by the so called "chain shuffling" technique, ie. the reassortment of a library of one chain type eg. heavy chain, with a fixed complementary chain, such as light chain, have also been described (Kang *et al*, 1991; Hoogenboom *et al*, 1991; Marks *et al*, 1992).

Our earlier International Patent Application No. PCT/AU93/00491 described recombinant constructs encoding target polypeptides having a stable core polypeptide region and at least one target-binding region, in which the target binding region(s) is/are covalently attached to the stable core polypeptide region, and has optionally been subjected to a maturation step to modify the specificity, affinity or avidity of binding to the target. The polypeptides may self-associate to form stable dimers, aggregates or arrays. The entire disclosure of PCT/AU93/00491 is incorporated herein by this cross-reference.

This specification did not predict that scFv-0 constructs in which the C-terminus of one V domain is ligated to the N-terminus of another domain, and therefore lack a foreign linker polypeptide, would form trimers. In contrast, it was suggested that, like constructs incorporating a linker, they would form dimers. A trimeric Fab' fragment formed by chemical means using a tri-maleimide cross-linking agent, referred to as tri-Fab, has been described (Schott *et al*, 1993 and Antoniw *et al*, 1996). These tri-Fab molecules, also termed TMF, have been labelled with ⁹⁰Y as potential agents for radioimmunotherapy of colon carcinoma, and have been shown to have superior therapeutic effects and fewer side-effects compared to the corresponding IgG. This was thought to result from more rapid penetration into the tumour and more rapid blood clearance, possibly resulting from the nature of the cross-linked antibody fragment rather than merely the lower molecular weight (Antoniw *et al*, 1996). However, these authors did not examine the affinity or avidity of either the IgG or the TMF construct.

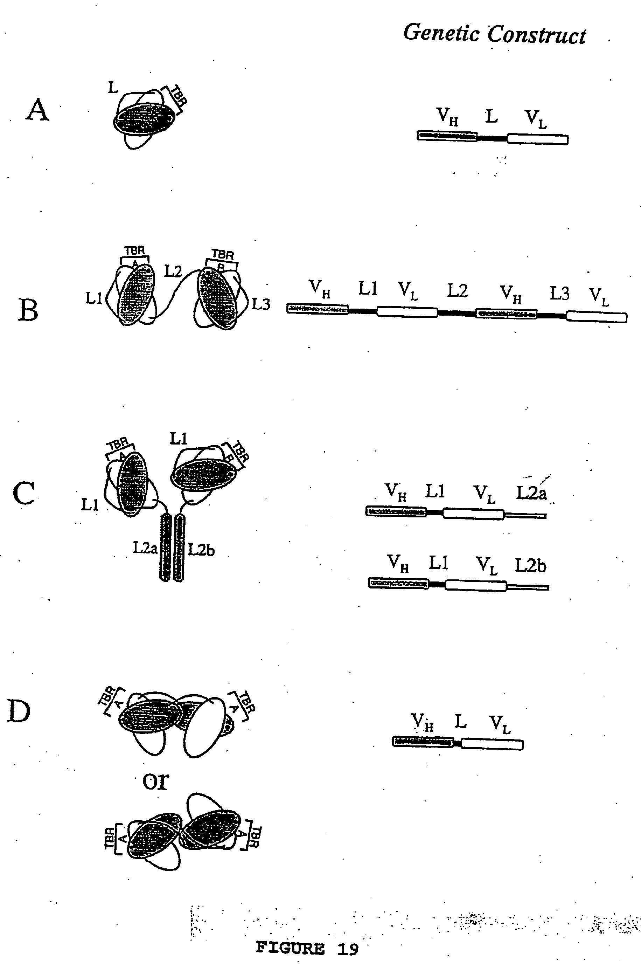

Recombinant single chain variable fragments (scFvs) of antibodies, in which the two variable domains V_{H} and V_{L} are covalently joined via a flexible peptide linker, have been shown to fold in the same conformation as the parent Fab (Kortt *et al*, 1994; Zdanov *et al*, 1994;see Figure 19a). ScFvs with linkers greater than 12 residues can form either stable monomers or dimers, and usually show the same binding specificity and affinity as the monomeric-form of the parent antibody (WO 31789/93, Bedzyk *et al*, 1990; Pantoliano *et al*, 1991), and exhibit improved stability compared to Fv fragments, which are not associated by covalent bonds and may dissociate at low protein concentrations (Glockshuber *et al*, 1990). ScFv fragments have been secreted as soluble, active proteins into the periplasmic space of *E*. *coli* (Glockshuber *et al*, 1990; Anand *et al*, 1991).

Various protein linking strategies have been used to produce bivalent or bispecific scFvs as well as bifunctional scFv fusions, and these reagents have numerous applications in clinical diagnosis and therapy (see Figure 19b-d). The linking strategies include the introduction of cysteine residues into a scFv monomer, followed by disulfide linkage to join two scFvs (Cumber *et al*, 1992; Adams *et al*, 1993; Kipriyanov *et al*, 1994; McCartney *et al*, 1995). Linkage between a pair of scFv molecules can also be achieved via a third polypeptide linker (Gruber *et al*, 1994; Mack *et al*, 1995; Neri *et al*, 1995;Figure 19b). Bispecific or bivalent scFv dimers have also been formed using the dimerisation properties of the kappa light chain constant domain (McGregor *et al*, 1994), and domains such as leucine zippers and four helix-bundles (Pack and Pluckthun, 1992; Pack *et al*, 1993, 1995; Mallender and Voss, 1994; Figure 19c). Trimerization of polypeptides for the association of immunoglobulin domains has also been described (International Patent Publication No. WO 95/31540). Bifunctional scFv fusion proteins have been constructed by attaching molecular ligands such as peptide epitopes for diagnostic applications (International Patent Application No. PCT/AU93/00228 by Agen Limited; Lilley *et al*, 1994; Coia *et al*, 1996), enzymes (Wels *et al*, 1992; Ducancel *et al*, 1993), streptavidin (Dubel *et al*, 1995), or toxins (Chaudhary *et al*, 1989, 1990; Batra *et al*, 1992; Buchner *et al*, 1992) for therapeutic applications.

In the design of scFvs, peptide linkers have been engineered to bridge the 35 Å distance between the carboxy terminus of one domain and the amino terminus of the other domain without affecting the ability of the domains to fold and form an intact binding site (Bird *et al*, 1988; Huston *et al*, 1988). The length and composition of various linkers have been investigated (Huston *et al*, 1991) and linkers of 14-25 residues have been routinely used in over 30 different scFv constructions, (WO 31789/93, Bird *et al*, 1988; Huston *et al*, 1988; Whitlow and Filpula, 1991; PCT/AU93/00491; Whitlow *et al*, 1993, 1994). The most frequently used linker is that of 15 residues (Gly₄Ser)₃ introduced by Huston *et al* (1988), with the serine residue enhancing the hydrophilicity of the peptide backbone to allow hydrogen bonding to solvent molecules, and the glycyl residues to provide the linker with flexibility to adopt a range of conformations (Argos, 1990). These properties also prevent interaction of the linker peptide with the hydrophobic interface of the individual domains. Whitlow *et al* (1993) have suggested that scFvs with linkers longer than 15 residues show higher affinities. In addition, linkers based on natural linker peptides, such as the 28 residue interdomain peptide of *Trichoderma reesi* cellobiohydrolase I, have been used to link the V_{H} and V_{L} domains (Takkinen *et al*, 1991).

A scFv fragment of antibody NC10 which recognises a dominant epitope of N9 neuraminidase, a surface glycoprotein of influenza virus, has been constructed and expressed in *E*. *coli* (PCT/AU93/00491; Malby *et al*, 1993). In this scFv, the V_{H} and V_{L} domains were linked with a classical 15 residue linker, (Gly₄ Ser)₃, and the construct contained a hydrophilic octapeptide (FLAG^{™}) attached to the C-terminus of the V_{L} chain as a label for identification and affinity purification (Hopp *et al*, 1988). The scFv-15 was isolated as a monomer which formed relatively stable dimers and higher molecular mass multimers on freezing at high protein concentrations. The dimers were active, shown to be bivalent (Kortt *et al*, 1994), and reacted with soluble N9 neuraminidase tetramers to yield a complex with an Mᵣ of - 600 kDa, consistent with 4 scFvs dimers cross-linking two neuraminidase molecules. Crystallographic studies on the NC10 scFv-15 monomer-neuraminidase complex showed that there were two scFv-neuraminidase complexes in the asymmetric unit and that the C-terminal ends of two V_{H} domains of the scFv molecules were in close contact (Kortt *et al*, 1994). This packing indicated that V_{H} and V_{L} domains could be joined with shorter linkers to form stable dimeric structures with domains pairing from different molecules and thus provide a mechanism for the construction of bispecific molecules (WO 94/13804, PCT/AU93/00491; Hudson et *al,* 1994, 1995).

Reduction of the linker length to shorter than 12 residues prevents the monomeric configuration and forces two scFv molecules into a dimeric conformation, termed diabodies (Holliger *et al*, 1993, 1996; Hudson *et al*, 1995; Atwell *et al*, 1996;Figure 19d). The higher avidity of these bivalent scFv dimers offers advantages for tumour imaging, diagnosis and therapy (Wu *et al*,. 1996). Bispecific diabodies have been produced using bicistronic vectors to express two different scFv molecules *in situ,* V_{H}A-linker-V_{L}B and V_{H}B-linker-V_{L}A, which associate to form the parent specificities of A and B (WO 94/13804; WO 95/08577; Holliger *et al*, 1996; Carter, 1996; Atwell *et al*, 1996). The 5-residue linker sequence, Gly₄Ser, in some of these bispecific diabodies provided a flexible and hydrophilic linker.

ScFv-0 V_{H}-V_{L} molecules have been designed without a linker polypeptide, by direct ligation of the C-terminal residue of V_{H} to the N-terminal residue of V_{L} (Holliger *et al*, 1993, McGuiness *et al*, 1996). These scFv-0 structures have previously been thought to be dimers.

We have now discovered that NC10 scFv molecules with V_{H} and V_{L} domains either joined directly together or joined with one or two residues in the linker polypeptide can be directed to form polyvalent molecules larger than dimers and in one aspect of the invention with a preference to form trimers. We have discovered that the trimers are trivalent, with 3 active antigen-combining sites (TBRs; target-binding regions). We have also discovered that NC10 scFv molecules with V_{L} domains directly linked to V_{H} domains can form tetramers that are tetravalent, with 4 active antigen-combining sites (TBRs).

We initially thought that these trimeric and tetrameric conformations might result from steric clashes between residues which were unique to the NC10scFv, and prevented the dimeric association. However, we have discovered that a second scFv with directly linked V_{H}-V_{L} domains, constructed from the monoclonal anti-idiotype antibody 11-1G10, is also a trimer and is trivalent, with 3 active TBRs. The parent antibody, murine 11-1G10, competes for binding to the murine NC41 antibody with the original target antigen, influenza virus N9 neuraminidase (NA) (Metzger and Webster, 1990). We have also discovered that another scFv with directly linked V_{H}-V_{L} domains (C215 specific for C215 antigen) is also a trimer.

We now propose that the propensity to form polyvalent molecules and particularly trimers is a general property of scFvs with V_{H} and V_{L} domains either joined directly together or joined with one or two residues in the linker polypeptide, perhaps due to the constraints imposed upon V-domain contacts for dimer formation. It will be appreciated by those skilled in the art that the polyvalent molecules can be readily separated and purified as trimers, tetramers and higher multimers.

Due to polyvalent binding to multiple antigens, trimers, tetramers and higher multimers exhibit a gain in functional affinity over the corresponding monomeric or dimeric molecules. This improved avidity makes the polymeric scFvs particularly attractive as therapeutic and diagnostic reagents. Furthermore the ability to utilise polycistronic expression vectors for recombinant production of these molecules enables polyspecific proteins to be produced.

### SUMMARY OF THE INVENTION

The invention generally provides polyvalent or polyspecific protein complexes, in which three or more polypeptides associate to form three or more functional target-binding regions (TBRs). A protein complex is defined as a stable association of several polypeptides via non-covalent interactions, and may include aligned V-domain surfaces typical of the Fv module of an antibody (Figure 1).

The individual polypeptides which form the polyvalent complex may be the same or different, and preferably each comprise at least two immunoglobulin-like domains of any member of the immunoglobulin superfamily, including but not limited to antibodies, T-cell receptor fragments, CD4, CD8, CD80, CD86, CD28 or CTLA4.

It will be clearly understood that the length of the linker joining the immunoglobulin-like domains on each individual polypeptide molecule is chosen so as to prevent the two domains from associating together to form a functional target-binding region (TBR) analogous to Fv, TCR or CD8 molecules. The length of the linker is also chosen to prevent the formation of diabodies. Instead, three or more separate polypeptide molecules associate together to form a polyvalent complex with three or more functional target-binding regions.

In a first aspect the invention provides a trimeric protein comprising three identical polypeptides, each of which comprises immunoglobulin V_{H} and V_{L} domains which are covalently joined preferably without a polypeptide linker, in which the peptides associate to form a trimer with three active TBRs, each of which is specific for the same target molecule.

In a second aspect the invention provides a trimeric protein comprising three different polypeptides, each of which comprises antibody V_{H} and V_{L} domains or other immunoglobulin domains, which are covalently joined preferably without a polypeptide linker, in which the polypeptides associate to form a trimer with three active TBRs directed against three different targets.

In one preferred embodiment of the second aspect the trimer comprises one TBR directed to a cancer cell-surface molecule and one or two TBRs directed to T-cell surface molecules.

In a second preferred embodiment the trimer comprises one TBR directed against a cancer cell surface molecule (a tumour antigen), and a second TBR directed against a different cell surface molecule on the same cancer cell.

In a third preferred embodiment the trimer comprises two TBRs directed against the same cancer cell-surface molecule and one TBR directed to a T-cell surface molecule.

In one preferred embodiment of the second aspect, one TBR of the trimer can be directed to a costimulatory T-cell surface molecule, such as CTLA4, CD28, CD80 or CD86.

Particularly preferred trivalent or trispecific reagents according to the invention include the following:
1) Three identical V_{H}-V_{L} molecules (scFv x 3)which are inactive as monomers but which form active trimers with 3 (identical) antigen combining sites (TBRs).
2) Three different V_{H}-V_{L} molecules (scFv x 3) which are inactive as monomers but which form active trimers with 3 different antigen combining sites (TBRs).

In a third aspect the invention provides a tetrameric protein comprising four identical polypeptides, each of which comprises immunoglobulin V_{H} and V_{L} domains which are covalently joined preferably without a polypeptide linker, in which the peptides associate to form a tetramer with four active TBRs each with specificity to the same target molecule.

In a fourth aspect the invention provides a tetrameric protein comprising four different polypeptides each of which comprises antibody V_{H} and V_{L} domains or other immunoglobulin domains, which are covalently joined preferably without a polypeptide linker, in which the polypeptides associate to form a tetramer with four active TBRs directed against four different targets.

In one preferred embodiment of the fourth aspect the tetramer comprises one or more TBRs directed to a cancer cell-surface molecule and one or more TBRs directed to T-cell surface molecules.

In a second preferred embodiment the tetramer comprises one or more TBRs directed against a cancer cell surface molecule (a tumour antigen), and one or more TBRs directed against a different cell surface molecule on the same cancer cell.

In one preferred embodiment of the fourth aspect, one TBR of the tetramer is directed to a costimulatory T-cell surface molecule, such as CTLA4, CD28, CD80 or CD86.

It will be clearly understood that the molecules which form the polyvalent or polyspecific proteins of the invention may comprise modifications introduced by any suitable method. For example one or more of the polypeptides may be linked to a biologically-active substance, chemical agent, peptide, drug or protein, or may be modified by site-directed or random mutagenesis, in order to modulate the binding properties, stability, biological activity or pharmacokinetic properties of the molecule or of the construct as a whole. The linking may be effected by any suitable chemical means alternatively, where the protein of the invention is to be conjugated to another protein or to a peptide, this may be achieved by recombinant means to express a suitable fusion protein. It will also be appreciated that chemical modifications and disulphide bonds to effect interdomain cross-links may be introduced in order to improve stability. Selection strategies may be used to identify desirable variants generated using such methods of modification. For example, phage display methods and affinity selection are very well known, and are widely used in the art.

Mechanisms to increase diversity and to select specific antibodies by the so-called "chain shuffling" technique, *ie*. the reassortment of a library of one chain type eg. heavy chain, with a fixed complementary chain, such as light chain, have also been described (Kang *et al,* 1991; Hoogenboom *et al*, 1991; Marks *et al*, 1992; Figini *et al*, 1994).

In order to avoid the generation of an immune response in the subject to which the polyvalent reagent of the invention is administered, and to ensure that repeat treatment is possible, it is preferred that the molecules comprising the polyvalent reagent are of homologous origin to the subject to be treated, or have been modified to remove as far as possible any xenoantigens. Thus if the recipient is a human, the molecules will be of human origin or will be humanised (CDR-grafted) versions of such molecules. "Humanisation" of recombinant antibody by grafting of CDRs is disclosed by Winter *et al*, EP-239400, and other appropriate methods, eg epitope imprinted selection (Figini *et al*, 1994), are also widely known in the art.

Where the immunoglobulin-like domains are derived from an antibody, the TBR may be directed to a chemical entity of any type. For example it may be directed to a small molecule such as a pesticide or a drug, a hormone such as a steroid, an amino acid, a peptide or a polypeptide; an antigen, such as a bacterial, viral or cell surface antigen; another antibody or another member of the immunoglobulin superfamily; a tumour marker, a growth factor etc. The person skilled in the art will readily be able to select the most suitable antigen or epitope for the desired purpose.

According to a fifth aspect, the invention provides a pharmaceutical composition comprising a polyvalent or polyspecific reagent according to the invention together with a pharmaceutically-acceptable carrier.

According to a sixth aspect the invention provides a method of treatment of a pathological condition, comprising the step of administering an effective amount of a polyvalent or polyspecific reagent according to the invention to a subject in need of such treatment, wherein one or more TBR of the reagent is directed to a marker which is:
a) characteristic of an organism which causes the pathological condition, or
b) characteristic of a cell of the subject which manifests the pathological condition, and another TBR of the reagent binds specifically to a therapeutic agent suitable for treatment of the pathological condition.

Preferably two different TBRs of the reagent are directed against markers of the pathological condition, and the third to the therapeutic agent, or alternatively one TBR of the reagent is directed to a marker for the pathological condition or its causative organism, and the two remaining TBRs of the reagent are directed to two different therapeutic agents. It is contemplated that the method of the invention is particularly suitable for treatment of tumours, in which case suitable therapeutic agents include but are not limited to cytotoxic agents, toxins and radioisotopes.

According to a seventh aspect the invention provides a method of diagnosis of a pathological condition, comprising the steps of administering a polyvalent or polyspecific reagent according to the invention to a subject suspected of suffering from said pathological condition, and identifying a site of localisation of the polyvalent or polyspecific reagent using a suitable detection method.

This diagnostic method of the invention may be applied to a variety of techniques, including radio imaging and dye marker techniques, and is suitable for detection and localisation of cancers, blood clots etc.

In another preferred embodiment of this aspect of the invention there is provided an imaging reagent comprising:
a) a trimer of the invention in which all three components (TBRs) of the trimer are directed to a molecular marker specific for a pathological condition and in which the trimer is either labelled with radioisotopes or is conjugated to a suitable imaging reagent.
b) a trimer of the invention in which either two TBRs of the trimer are directed to two different markers specific for a pathological condition or site, and the third is directed to a suitable imaging reagent;
c) one TBR of the trimer is directed to a marker characteristic of a pathological condition, such as a tumour marker, a second TBR is directed to a marker specific for a tissue site where the pathological condition is suspected to exist, and the third is directed to a suitable imaging agent, or
d) one TBR of the trimer is directed to a marker characteristic of the pathological condition and the remaining two TBRs are directed to two different imaging agents.

In one preferred embodiment of the invention, one component of the polyspecific molecule is a non-antibody immunoglobulin-like molecule. These Ig-like molecules are useful for binding to cell surfaces and for recruitment of antigen presenting cells, T-cells, macrophages or NK cells. The range of Ig-like molecules for these applications includes:
a) The Ig-like extracellular domain of CD28 and derivatives or CTLA4 and derivatives (Linsley *et al,* 1995). CTLA4 binds to its cognate receptors B7-1 and B7-2 on antigen presenting cells, either as a monomer (a single V-like domain) or as a dimer or as a single chain derivative of a dimer.
b) The Ig-like extracellular domains of B7-1 and B7-2 (CD80, CD86 respectively; Peach *et al,* 1995, Linsley *et al,* 1994) which have homology to Ig variable and constant domains.

In a preferred embodiment, the Ig-like domains described above are affinity-matured analogues of the natural mammalian sequence which have been selected to possess higher binding affinity to their cognate receptor than that of the natural sequence. Techniques for affinity maturation are well known in the field, and include mutagenesis of CDR-like loops, framework or surface regions and random mutagenesis strategies (Irving *et al,* 1996). Phage display can be used to screen a large number of mutants (Irving *et al,* 1996). CTLA4 and CD80/86 derivatives with enhanced binding activity (through increases in functional affinity) have application in preventing transplant rejection and intervening in autoimmune diseases. These molecules interfere with T-cell communication to antigen presenting cells, and can either activate T-cells leading to proliferation with application as an anti-cancer reagent, or decrease T-cell activation, leading to tolerance, with application in the treatment of autoimmune disease and transplantation (Linsley *et al,* 1994,1995). These molecules can also be used to activate NK cells and macrophages once recruited to a target site or cell population.

In a further preferred embodiment, trispecific reagents comprise dimeric versions of CTLA4 or CD80/86 or one molecule of each species, which is expected to result in further affinity enhancement and with similar therapeutic applications as described above.

In a further preferred embodiment, one component of the trispecific reagents may comprise a non Ig-like domains, such as CD40, to manipulate the activity of T and NK cells.

### Brief Description of the Figures

Figure 1 shows a schematic representation of some polyvalent and/or polyspecific antibody proteins and protein complexes. * Indicates configurations for which the design has been described in this specification. Ovals represent Ig V and C domains, and the dot in the V-domain represents the N-terminal end of the domain. Ovals which touch edge-to-edge are covalently joined together as a single polypeptide, whereas ovals which overlay on top of each other are not covalently joined. It will be appreciated that alternative orientations and associations of domains are possible.

Figure 1 also shows a schematic representation of intact IgG, and its Fab and Fv fragments, comprising V_{H} and V_{L} domains associated to form the TBR; for both the intact IgG and Fab the C_{H}1 and C_{L} domains are also shown as ovals which associate together. Also shown are Fab molecules conjugated into a polyvalent reagent either by Celltech's TFM chemical cross-linker or by fusion to amphipathic helices with adhere together. A monomeric scFv molecule is shown in which the V_{H} and V_{L} domains are joined by a linker of at least 12 residues (shown as a black line). Dimers are shown as bivalent scFv₂ (diabodies) with two identical V_{H}-L-V_{L} molecules associating to form two identical TBRs (A), and bispecific diabody structures are shown as the association of two V_{H}-L-V_{L} molecules to form two different TBRs (A,B) and where the polypeptide linker (L) is at least 4 residues in length. Aspect 1 of the invention is shown as a trivalent scFv₃ (triabody) in which three identical V_{H}-V_{L} molecules associate to form three identical TBRs (A) and where the V-domains are directly ligated together preferably without a polypeptide linker sequence. Aspect 2 of the invention is depicted as a trispecific triabody with association of three V_{H}-V_{L} molecules to form three different TBRs (A,B,C). Aspects 3,4 of the invention are shown as a tetravalent ScFv₄ tetramer (tetrabody) and a tetraspecific tetrabody with association of four identical or different scFv molecules respectively and in which the V-domains are directly ligated together preferably without a polypeptide linker sequence.

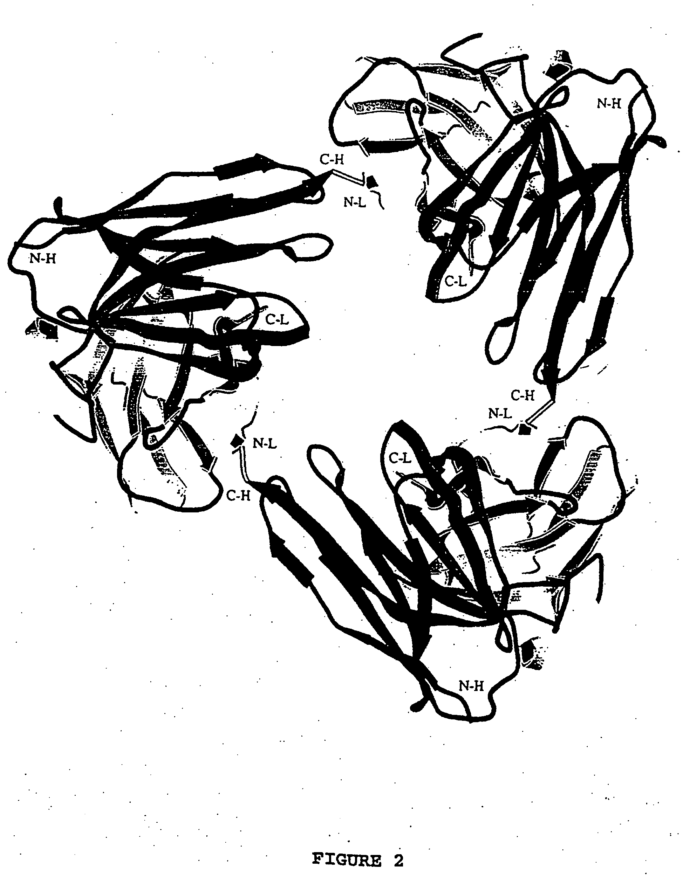

Figure 2 shows a ribbon structure model of the NC10 scFv-0 trimer constructed with circular three-fold symmetry. The three-fold axis is shown out of the page. The V_{H} and V_{L} domains are shaded dark grey and light grey, respectively. CDRs are shown in black, and the peptide bonds (zero residue linkers) joining the carboxy terminus of V_{H} to the amino terminus of the V_{L} in each single chain are shown with a double line. Amino (N) and carboxy (C) termini of the V_{H} (H) and V_{L} (L) domains are labelled.

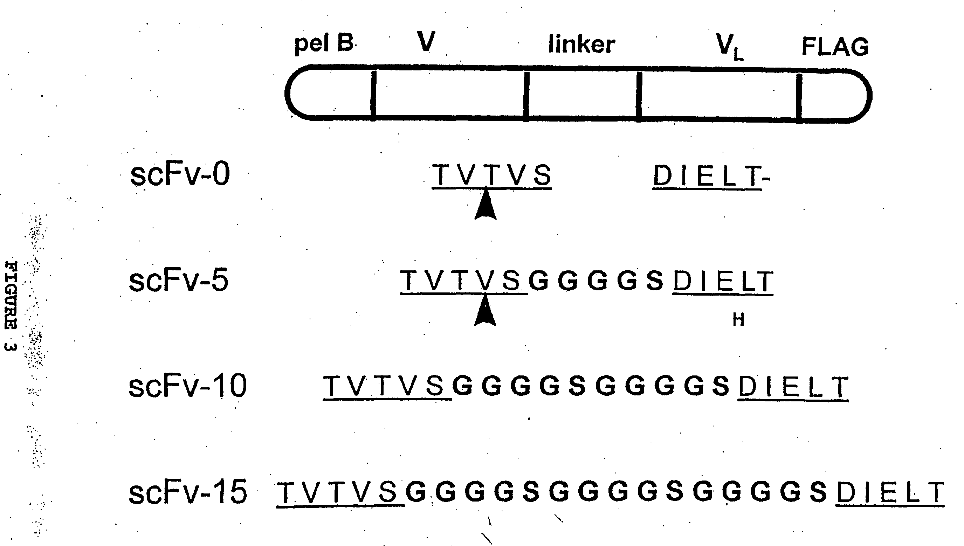

Figure 3 shows a schematic diagram of the scFv expression unit, showing the sequences of the C-terminus of the V_{H} domain (residues underlined), the N-terminus of the V_{L} domain (residues underlined) and of the linker peptide (bold) used in each of the NC10 scFv constructs.

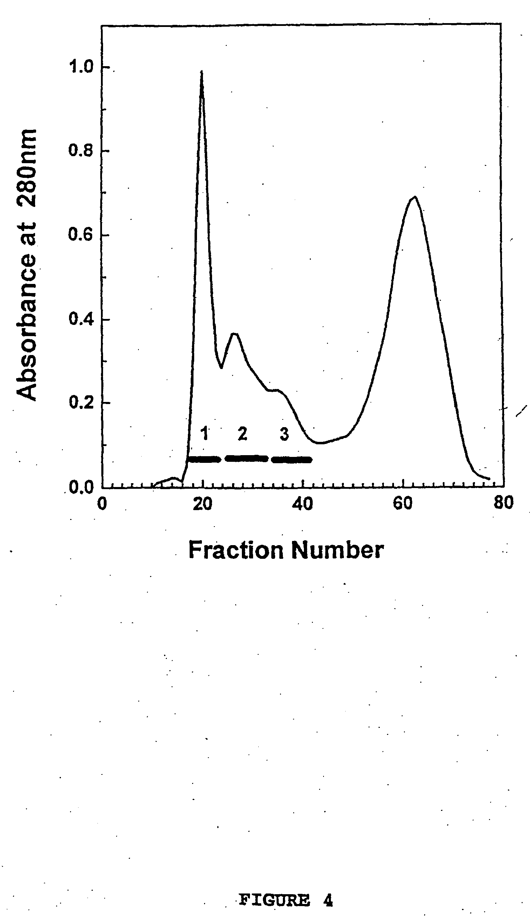

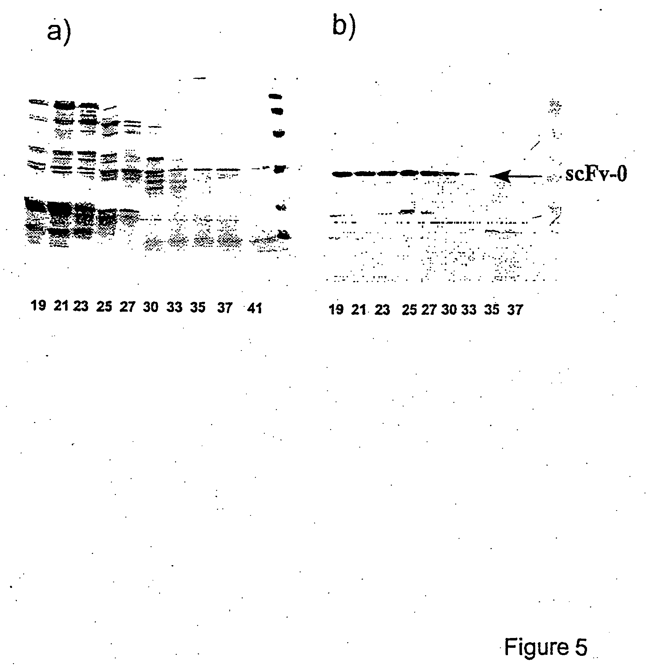

Figure 4 shows the results of Sephadex G-100 gel filtration of solubilised NC10 scFv-0 obtained by extraction of the insoluble protein aggregates with 6 M guanidine hydrochloride. The column (60 x 2.5 cm) was equilibrated with PBS, pH 7.4 and run at a flow rate of 40 ml/hr; 10 ml fractions were collected. Aliquots were taken across peaks 1-3 for SDS-PAGE analysis to locate the scFv using protein stain (Coomassie brilliant blue G-250) and Western blot analysis (see Figure 5). The peaks were pooled as indicated by the bars.

Figure 5 shows the results of SDS-PAGE analysis of fractions from the Sephadex G-100 gel filtration of scFv-0 shown in Figure 4. Fractions analysed from peaks 1-3 are indicated;
a) Gel stained with Coomassie brilliant blue G-250;
b) Western blot analysis of the same fractions using anti-FLAG^{™} M2 antibody.

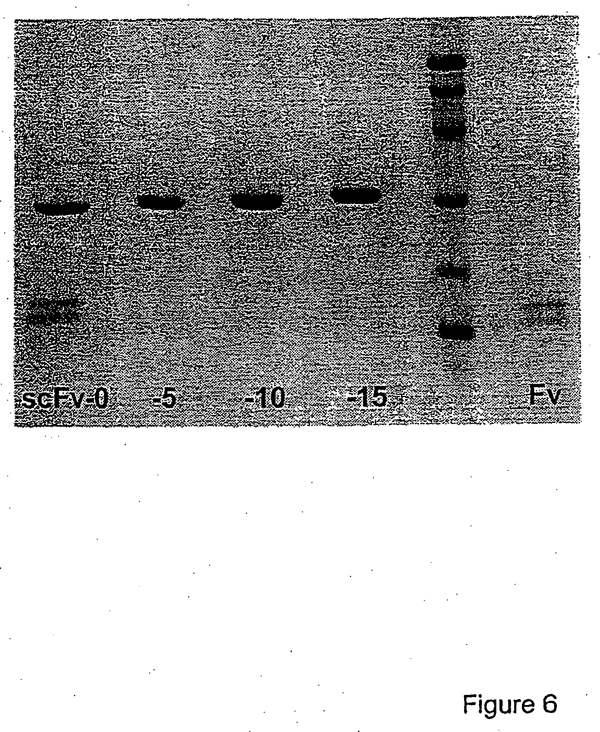

Figure 6 shows the results of SDS-PAGE comprising affinity-purified NC10 scFvs with the V_{H} and V_{L} domains joined by linkers of different lengths. ScFv-0 shows two lower molecular mass bands of -14 kDa and 15 kDa (arrowed), corresponding to the V_{H} and V_{L} domains produced by proteolytic cleavage of the scFvs during isolation, as described in the text. The far right lane shows the monomer peak (Fv) isolated from the scFv-0 preparation (left lane) by gel filtration.

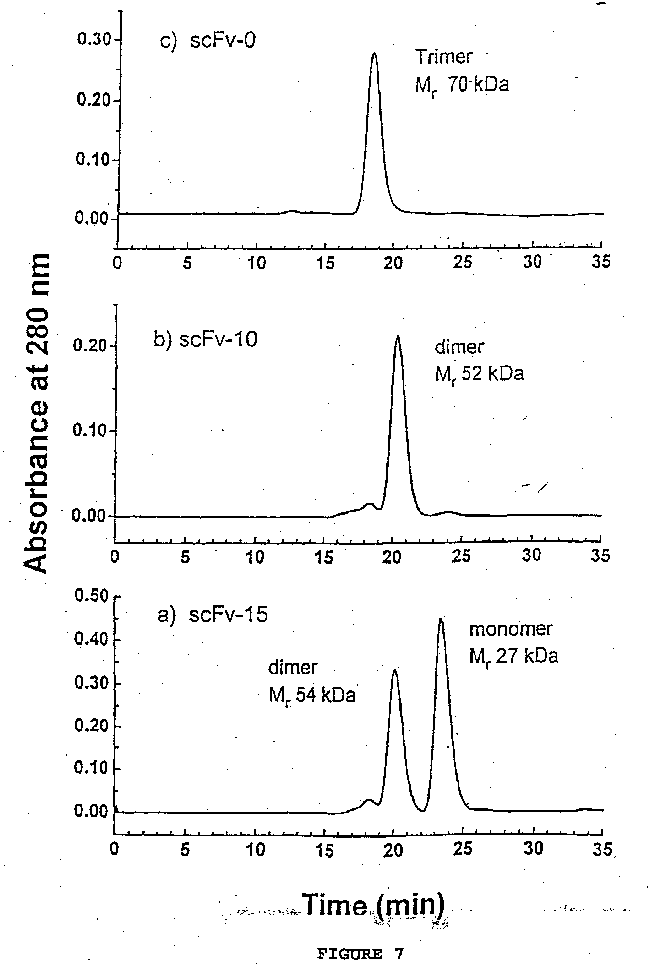

Figure 7 shows the results of size exclusion FPLC of affinity purified NC10 scFvs on a calibrated Superdex 75 HR10/30 column (Pharmacia). The column was calibrated as described previously (Kortt *et al*, 1994). Panel a shows that the scFv-15 contains monomer, dimer and some higher Mr multimers. Panel b shows the scFv-10, containing predominantly dimer, and Panel c shows the scFv-0 eluting as a single.peak with Mᵣ of - 70 kDa. The column was equilibrated with PBS, pH 7.4 and run at a flow rate of 0.5 ml/min.

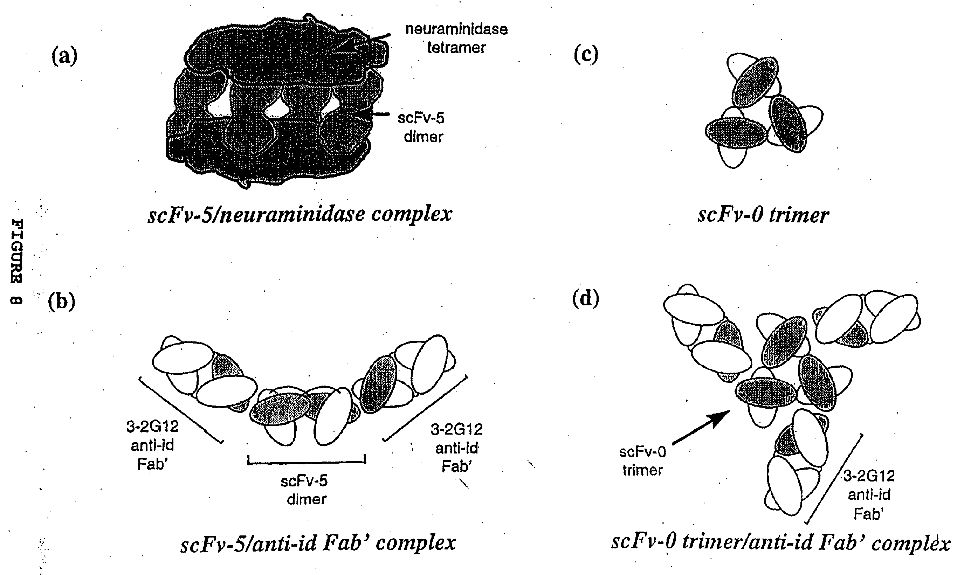

Figure 8 shows diagrams illustrating
a) the 'sandwich' complex between two tetrameric neuraminidases and four scFv dimers based on crystallographic data of the neuraminidase-Fab complex (Tulip *et al*, 1992; Malby *et al*, 1994) and scFv-15 monomer complex (Kortt *et al*, 1994),
b) the complex between scFv-5 dimer and anti-idiotype 3-2G12 Fab',
c) the scFv-0 trimer (c.f. Figure 2), and
d) the scFv-0 binding three anti-idiotype Fab' fragments to form a complex of Mᵣ 212 kDa.

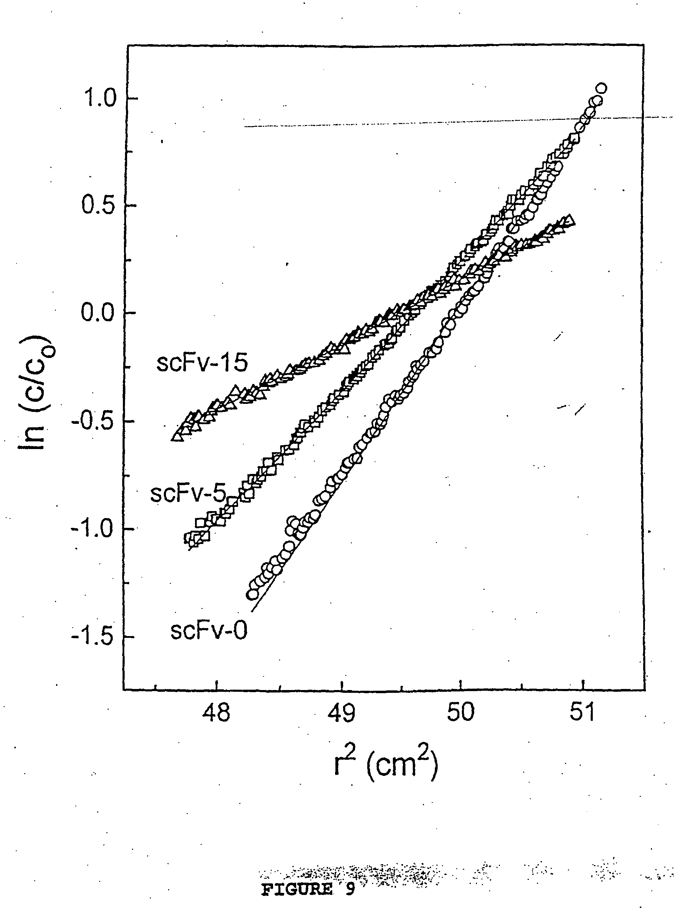

Figure 9 shows sedimentation equilibrium data for complexes of anti-idiotype 3-2G12 Fab' and NC10 scFv-15 monomer, scFv-5 dimer and scFv-0 trimer. The complexes were isolated by size exclusion chromatography on Superose 6 in 0.05 M sodium phosphate, 0.15 M NaCl, pH 7.4. Experiments were conducted at 1960 g at 20°C for 24 h using double sector centrepiece and 100 µl sample. The absorbance at 214 nm was determined as a function of radius in cm. Data for the complexes of anti-idiotype 3-2G12 Fab' with scFv-15 monomer (Δ), scFv-5 () and scFv-0 (0) are shown.

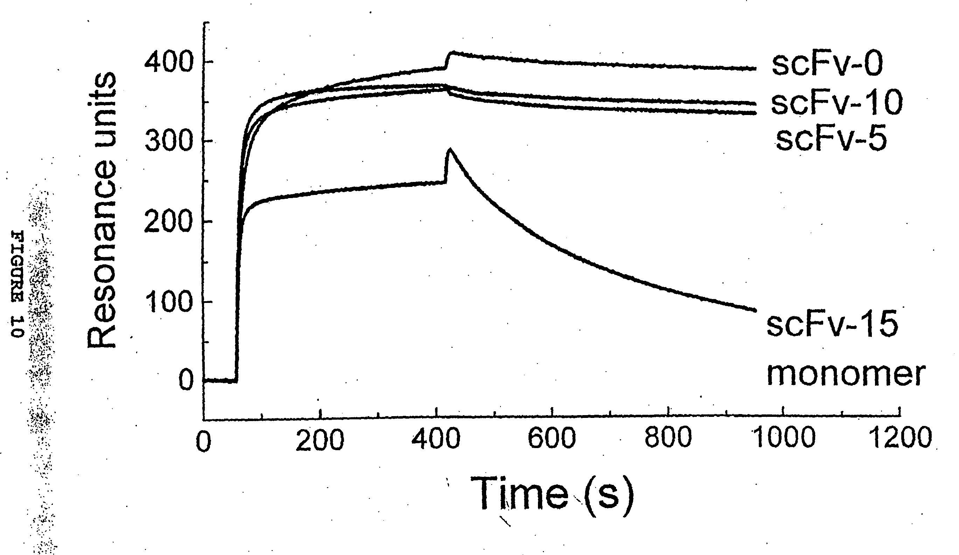

Figure 10 shows BIAcore^{™} biosensor sensorgrams demonstrating the binding of NC10 scFv-15 monomer, scFv-10 dimer, scFv-5 dimer and scFv-0 trimer, each at a concentration of 10 µg/ml, to immobilised anti-idiotype 3-2G12 Fab' (1000 RU). An injection volume of 30 µl and a flow rate of 5 µl/min were used. The surface was regenerated with 10 µl of 10 mM sodium acetate, pH 3.0 after each binding experiment.

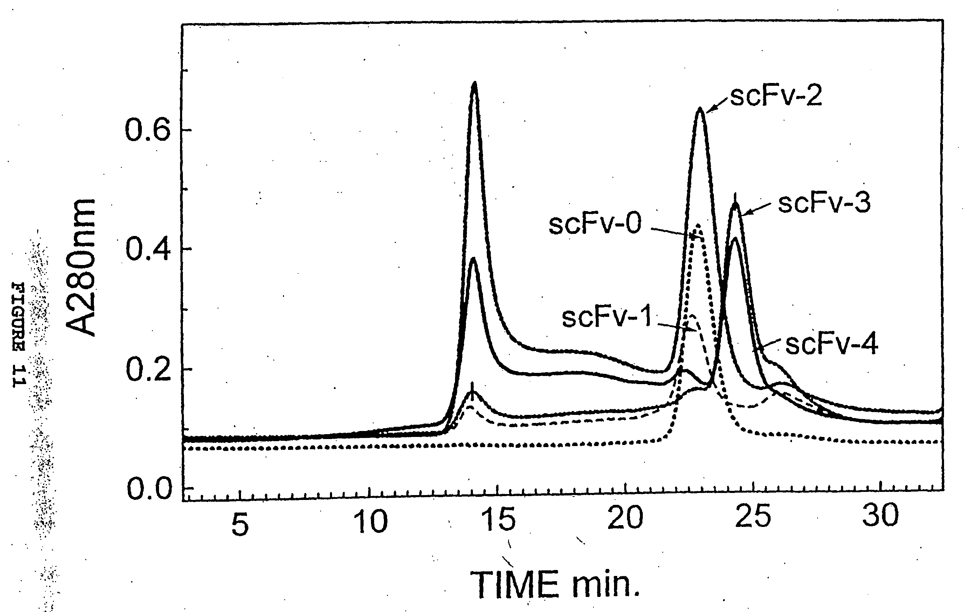

Figure 11 shows the results of size exclusion FPLC of affinity purified NC10 scFv-1, scFv-2, scFv-3 and scFv-4 on a calibrated Superose 12 column HR10/30 (Pharmacia). The results of four separate runs are superimposed. The column was equilibrated with PBS, pH7.4 and run at a flow rate of 0.5 ml/min

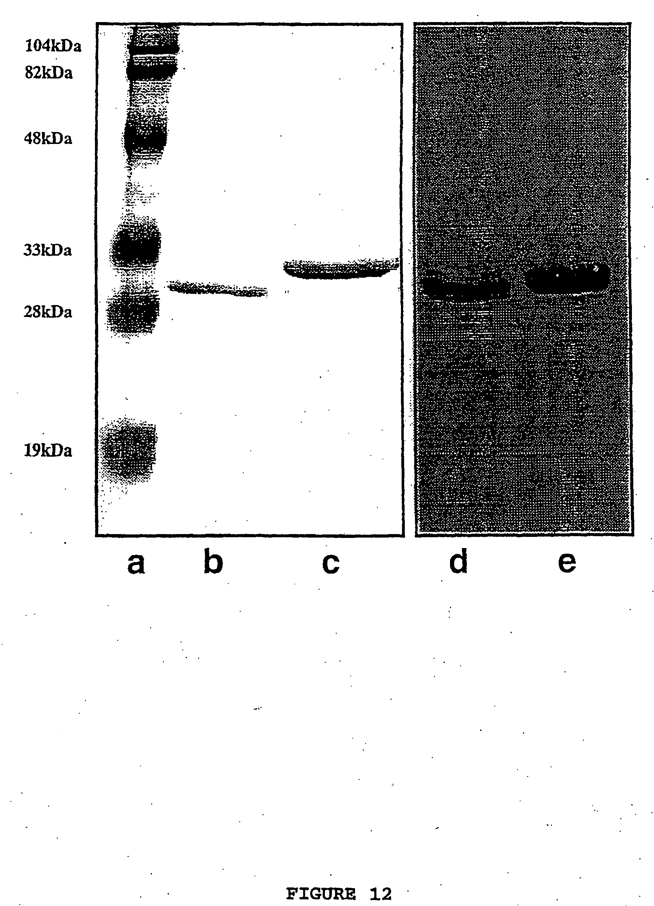

Figure 12 shows the results of SDS-PAGE analysis of 11-1G10 scFv-15 and 11-1G10 scFv-0 and Western Transfer detection using anti-FLAG M2 antibody; lanes on Coomassie stained gel (a) BioRad Low MW standards, (b) scFv-0, (c) scFv-15 and corresponding Western blot of (d) scFv-0 and (e) scFv-15. The theoretical MW of scFv-15 is 28427 Da and scFv-0 is 26466 Da.

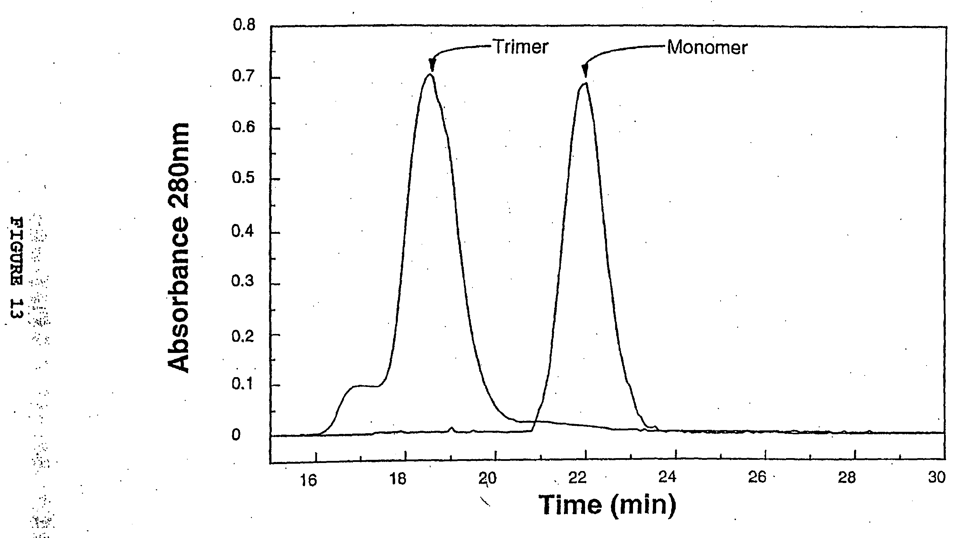

Figure 13 shows the results of size exclusion FPLC on a calibrated Superdex 75 HR10/30 column (Pharmacia), showing overlaid profiles of 11-1G10 scFv-15 monomer and scFv-0 trimer with peaks eluting at times corresponding to Mᵣ ∼27 kDa and ∼85kDa respectively. The column was equilibrated with PBS (pH 7.4) and run at a flow rate of 0.5 ml/min.

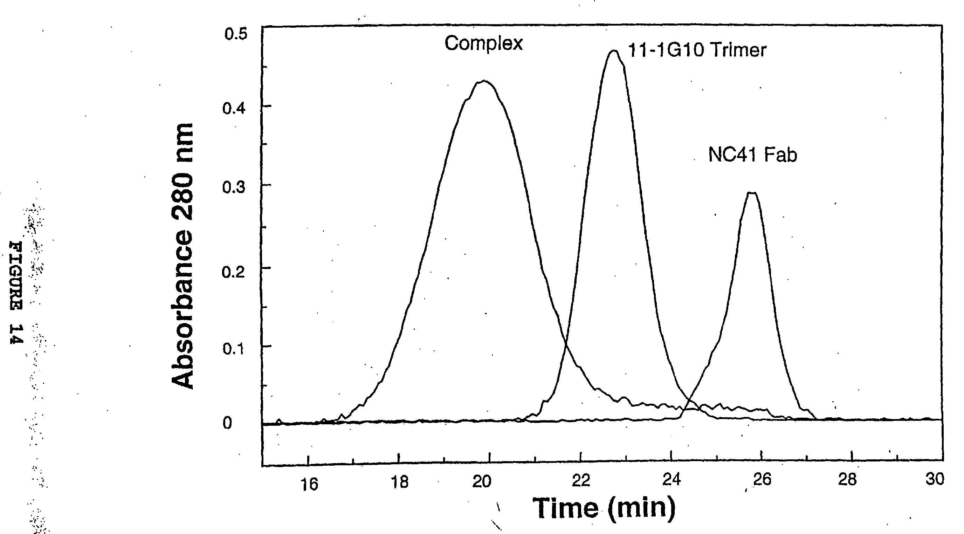

Figure 14 shows the results of size exclusion FPLC on a calibrated Superose 12 HR10/30 column (Pharmacia), showing overlaid profiles of the isolated 11-1G10 scFv-0 trimer, NC41 Fab and scFv/Fab complex formed on the interaction of scFv-0 and NC41 Fab premixed in 1:3 molar ratio. The column was equilibrated with PBS (pH 7.4) and run at a flow rate of 0.5 ml/min.

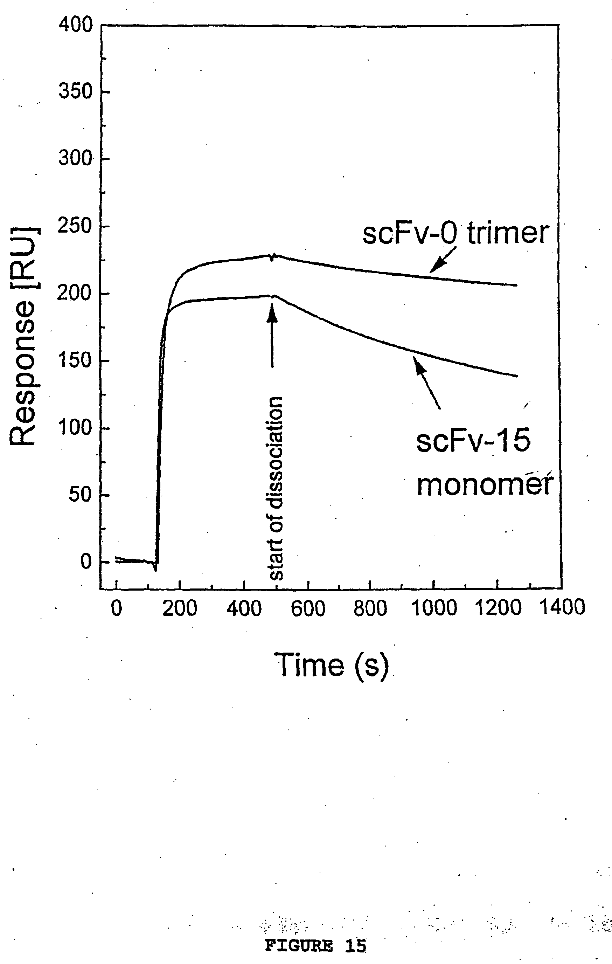

Figure 15 shows BIAcore^{™} biosensor sensorgrams showing the association and dissociation of 11-1G10 scFv-15 monomer and scFv-0 trimer, each at a concentration of 222 nM, to immobilised NC41 Fab. An injection volume of 30 µl and a flow rate of 5 µl/min were used. The surface was regenerated with 10 µl of 10 mM sodium acetate, pH 3.0 after each binding experiment.

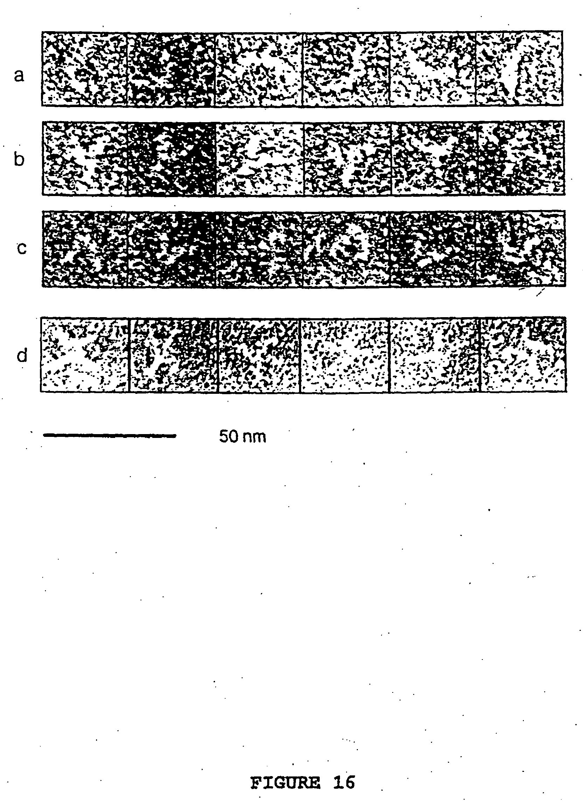

Figure 16 shows a gallery of selected particles from electron micrographs of
a) boomerangs; NC10 V_{H}-V_{L} scFv-5 diabody/ 3-2G12 Fab complex,
b) Y-shaped tripods; NC10 V_{H}-V_{L} scFv-0 triabody/3-2G12 Fab complex,
c) V-shaped projections; NC10 V_{H}-V_{L} scFv-0 triabody/ 3-2G12 Fab complex, and
d) X-shaped tetramers; NC10 V_{L}-V_{H} scFv-0 tetramer/ 3-2G12 Fab complex.
   Magnification bar 50nm.

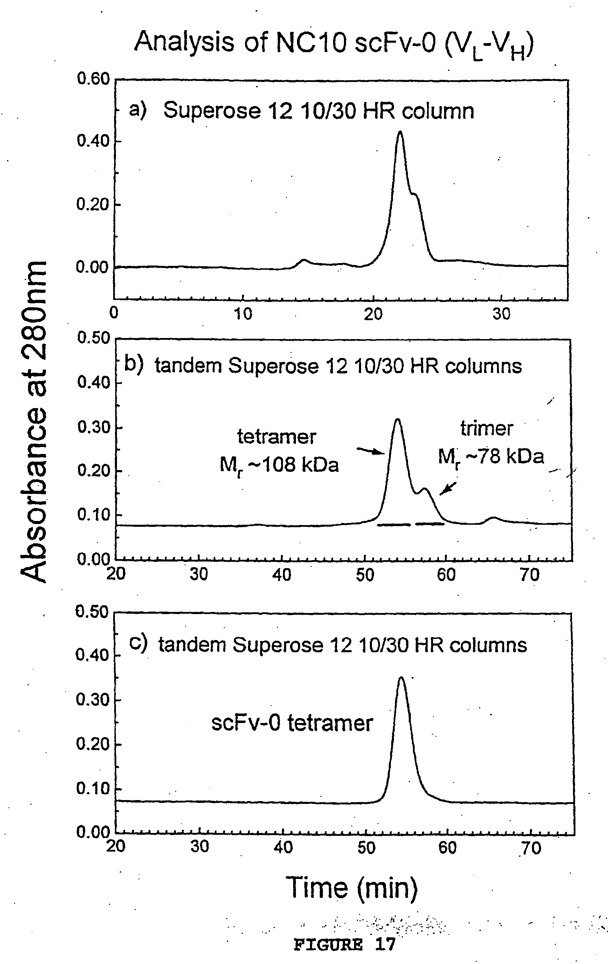

Figure 17 shows the analysis of affinity-purified NC10 scFv-0 (V_{L}-V_{H}) on a Superose 12 10/30 HR (Pharmacia) column. Panel a) shows the profile for the affinity purified scFv on a single Superose 12 column equilibrated in PBS pH 7.4 and run at a flow rate of 0.5 ml/min. The scFv-0 contains two components. Panel b) shows the separation of the two components in the affinity-purified scFv-0 preparation on two Superose 12 columns joined in tandem to yield a scFv-0 tetramer (Mᵣ ∼108 kDa) and a scFv-0 trimer (Mᵣ∼ 78 kDa). The tandem columns were equilibrated in PBS, pH 7.4 and run at a flow rate of 0.3 ml/min. The peaks were pooled as indicated by the bars for complex formation with 3-2G12 antibody Fab' used for EM imaging. Panel c) shows the profile for the rechromatography of the isolated scFv-0 tetramer from panel b on the tandem Superose columns under the conditions used in panel b.

Figure 18 shows the size exclusion FPLC analysis of affinity-purified C215 scFv-0 (V_{H}-V_{L}) on a Superose 12 10/30 HR column (Pharmacia) equilibrated in PBS pH 7.4 and run at a flow rate of 0.5 ml/min.

Figure 19 illustrates different types of scFv-type constructs of the prior art.
A: An scFv comprising V_{H}-L-V_{L} where L is a linker polypeptide as described by Whitlow et al and WO 93/31789; by Ladner et al, US-4,946,778 and WO 88/06630; and by McCafferty *et al* (1991) and by McCartney et *al*.(1995).
B: A single polypeptide V_{H}-L1-V_{L}-L2-V_{H}-L3-V_{L} which forms two scFv modules joined by linker polypeptide L2, and in which the V_{H} and V_{L} domains of each scFv module are joined by polypeptides L1 and L3 respectively. The design is described by Chang, AU-640863.
C: Two scFv molecules each comprising V_{H}-L1-V_{L}-L2(a,b), in which the V_{H} and V_{L} domains are joined by linker polypeptide L1 and the two scFv domains are joined together by a C-terminal adhesive linkers L2a and L2b. The design is described by Pack *et al*, PI-93-258685.
D: The design of PCT/AU93/00491, clearly different to A, B and C above. A single scFv molecule V_{H}-L-V_{L} comprises a shortened linker polypeptide L which specifically prevents formation of scFvs of the type A, B or C, and instead forces self-association of two scFvs into a bivalent scFv dimer with two antigen combining sites (target-binding regions; TBR-A). The association of two different scFv molecules will form a bispecific diabody (TBRs-A,B).

### DETAILED DESCRIPTION OF THE INVENTION

The invention will be described in detail by reference only to the following non-limiting examples and to the figures.

### General Materials and Methods

### Preparation of tern N9 neuraminidase and Fab fragments of anti-neuraminidase antibody NC41 and anti-idiotype antibodies 3-2G12 and 11-1G10

N9 neuraminidase was isolated from avian (tern) influenza virus following treatment of the virus with pronase and purified by gel filtration as described previously (McKimm-Breschkin *et al*, 1991).

Monoclonal anti-idiotype antibodies 3-2G12 and 11-1G10 were prepared in CAF1 mice against NC10 and NC41 anti-neuraminidase BALB/c monoclonal antibodies (Metzger and Webster, 1990). Anti-neuraminidase antibody NC41 and the anti-idiotype antibodies 3-2G12 and 11-1G10 were isolated from ascites fluid by protein A-Sepharose chromatography (Pharmacia Biotech). Purified antibodies were dialysed against 0.05 M Tris-HCl, 3 mM EDTA, pH 7.0 and digested with papain to yield F(ab')2 as described (Gruen *et al*, 1993). The F(ab')2 fragment from each antibody was separated from Fc and undigested IgG by chromatography on protein A-Sepharose, and pure F(ab')2 was reduced with 0.01 M mercaptoethylamine for 1h at 37° C and the reaction quenched with iodoacetic acid. The Fab' was separated from the reagents and unreduced F(ab')2 by gel filtration on a Superdex 75 column (HR 10/30) in PBS, 7.4.

### Size exclusion FPLC chromatography and molecular mass determination

The molecular size and aggregation state of affinity purified scFvs were assessed by size exclusion FPLC on Superose 6 or 12, or Superdex 75 HR 10/30 (Pharmacia) columns in PBS, pH 7.4. The ability of the scFv-0, scFv-5 and scFv-10 to bind to antigen and anti-idiotype Fab' fragments, and the size of the complexes formed, was also assessed by size exclusion FPLC on Superose 6 in PBS, pH 7.4. The columns were equilibrated with a set of standard proteins as described previously (Kortt *et al*, 1994).

The molecular mass of scFv-0, scFv-5 and scFv-10, and that of the complexes formed with antigen and anti-idiotype antibody Fab' fragments with each scFv, was determined in 0.05 M phosphate-0.15 M NaCl, pH 7.4 by sedimentation equilibrium in a Beckman model XLA ultracentrifuge.

### Biosensor binding analysis

The BIAcore^{™} biosensor (Pharmacia Biosensor AB, Uppsala Sweden), which uses surface plasmon resonance detection and permits real-time interaction analysis of two interacting species (Karlsson *et al*, 1991; Jonsson *et al,* 1993), was used to measure the binding kinetics of the different NC10 scFvs. Samples for binding analyses were prepared for each experiment by gel filtration on Superdex 75 or Superose 12 to remove any cleavage products or higher molecular mass aggregates which may have formed on storage. The kinetic constants, kₐ and k_{d}, were evaluated using the BIAevaluation 2.1 software supplied by the manufacturer, for binding data where the experimental design correlated with the simple 1:1 interaction model used for the analysis of BIAcore^{™} binding data (Karlsson *et al*, 1994).

### Electron microscopy

Solutions of the two complexes; NC10 scFv-5 diabody/Fab, NC10 scFv-0 triabody/Fab, and also a mixture of NC10 scFv-0 triabody/Fab with free 3-G12 anti-idiotype Fab were examined by electron microscopy. In each case, proteins were diluted in phosphate-buffered saline (PBS) to concentrations of the order of 0.01-0.03 mg/ml. Prior to dilution, 10% glutaraldehyde (Fluka) was added to the PBS to achieve a final concentration of 1% glutaraldehyde. Droplets of - 3 µl of this solution were applied to thin carbon film on 700-mesh gold grids which had been glow-discharged in nitrogen for 30 s. After 1 min the excess protein solution was drawn off, followed by application and withdrawal of 4-5 droplets of negative stain (2% potassium phosphotungstate adjusted to pH 6.0 with KOH). The grids were air-dried and then examined at 60 kV in a JEOL 100B transmission electron microscope at a magnification of 100,000x. Electron micrographs were recorded on Kodak SO-163 film and developed in undiluted Kodak D19 developer. The electron-optical magnification was calibrated under identical imaging conditions by recording single-molecule images of the NC10 antibody (Fab) complex with its antigen, influenza virus neuraminidase heads.

Measurements of particle dimensions were made on digitised micrographs using the interactive facilities of the SPIDER image processing suite to record the coordinates of particle vertices. Particle arm lengths and inter-arm angles were calculated from the coordinates for 229 diabodies and 114 triabodies.

### Example 1 Construction of NC10 scFv (V_{H}-V_{L}) with 0, 5 and 10 Residue Linkers

The NC10 scFv antibody gene construct with a 15 residue linker (Malby *et al*, 1993) was used for the shorter linker constructions. The NC10 scFv-15 gene was digested successively with *Bst*EII (New England Labs) and SacI (Pharmacia) and the polypeptide linker sequence released. The remaining plasmid which contained NC10 scFv DNA fragments was purified on an agarose gel and the DNA concentrated by precipitation with ethanol. Synthetic oligonucleotides (Table 1) were phosphorylated at the 5' termini by incubation at 37°C for 30 min with 0.5 units of T4 polynucleotide kinase (Pharmacia) and 1 mM ATP in One-Phor-All buffer (Pharmacia). Pairs of complementary phosphorylated oligonucleotide primers (Table 1) were premixed in equimolar ratios to form DNA duplexes which encoded single chain linkers of altered lengths.

**Table 1**

| DNA Sequences of Synthetic Oligonucleotide Duplexes Encoding Peptide Linkers of Different Lengths Inserted Into the *Bst*EII and *Sac*I Restriction Sites of pPOW-scFv NC10 (between the carboxyl of the V_{H} and the amino terminal of V_{L}) | | | | | | | | | | | | | | | | | | | | | | | | | | |
|---|---|---|---|---|---|---|---|---|---|---|---|---|---|---|---|---|---|---|---|---|---|---|---|---|---|---|
| Construct | Complementary Oligonucleotide Pair | | | | | | | | | | | | | | | | | | | | | | | | | SEQ ID NO. |
| scFv-15 | 5' GIC ACC GTC TCC GGT GGT GGT GGT TCG GGT GGT GGT GGT TCG GGT GGT GGT GGT TCG GAT ATC GAG CT 3' | | | | | | | | | | | | | | | | | | | | | | | | | 1 |
| | 3' G CAG AGG CCA CCA CCA CCA AGC CCA CCA CCA CCA AGC CCA CCA CCA CCA AGC CTA TAG C 5' | | | | | | | | | | | | | | | | | | | | | | | | | 2 |
| scFv-10 | 5' GTC ACC GTC TCC GGT GGT GGT GGT TCG GGT GGT GGT GGT TCG GAT ATC GAG CT 3' | | | | | | | | | | | | | | | | | | | | | | | | | 3 |
| | 3' G CAG AGG CCA CCA CCA CCA AGC CCA CCA CCA CCA AGC CTA TAG C 5' | | | | | | | | | | | | | | | | | | | | | | | | | 4 |
| scFv-5 | 5' GTC ACC GTC TCC GGT GGT GGT GGT TCG GAT ATC GAG CT 3' | | | | | | | | | | | | | | | | | | | | | | | | | 5 |
| | 3' G CAG AGG CCA CCA CCA CCA AGC CTA TAG C 5' | | | | | | | | | | | | | | | | | | | | | | | | | 6 |
| sCFv-0 | 5' GIC ACC GTC TCC GAT ATC GAG CT 3' | | | | | | | | | | | | | | | | | | | | | | | | | 7 |
| | 3' G CAG AGG CTA TAG C 5' | | | | | | | | | | | | | | | | | | | | | | | | | 8 |

These duplexes were ligated into BstEII-SacI restricted pPOW NC10 scFv plasmid using an Amersham ligation kit. The ligation mixture was purified by phenol/chloroform extraction, precipitated with ethanol in the usual manner, and transformed into *E*. *coli* DH5α (*sup*E44, *hsd*R17, *rec*A1, *end*A1, *gyr*A96, *thi*-1, *rel*A1) and LE392 (*sup*E44, *sup*F58, *hsd*R14, *lac*Y1, *gal*K2, *gal*T22, *met*B1, *trp*R55). Recombinant clones were identified by PCR screening with oligonucleotides directed to the pelB leader and FLAG sequences of the pPOW vector. The DNA sequences of the shortened linker regions were verified by sequencing double-stranded DNA using Sequenase 2.0 (USB).

The new NC10 scFv gene constructs, in which the V_{H} and V_{L} domains were linked with linkers of 10 ((Gly₄Ser)₂), 5 (Gly₄Ser) and zero residues, are shown in Figure 3. DNA sequencing of the new constructs confirmed that there were no mutations, and that the V_{H} and V_{L} domains were joined by the shorter linker lengths as designed. These constructs are referred to herein as NC10 scFv-10, scFv-5 and scFv-0, where the number refers to the number of residues in the linker.

### Example 2 Expression and purification of the NC10 scFvs

The pPOW NC10 scFv constructs, with 0, 5 and 10 residues linkers as described in Example 1, were expressed as described by Malby *et al*, (1993) for the parent scFv-15. The protein was located in the periplasm as insoluble protein aggregates associated with the bacterial membrane fraction, as found for the NC10 scFv-15 (Kortt *et al,* 1994). Expressed NC10 scFvs with the shorter linkers were solubilised in 6M guanidine hydrochloride/0.1 M Tris/HCl, pH 8.0, dialysed against PBS, pH 7.4 and the insoluble material was removed by centrifugation. The soluble fraction was concentrated approximately 10-fold by ultrafiltration (Amicon stirred cell, YM10 membrane) as described previously (Kortt *et al*, 1994) and the concentrate was applied to a Sephadex G-100 column (60 x 2.5 cm) equilibrated with PBS, pH 7.4; fractions which contained protein were analysed by SDS-PAGE and the scFv was located by Western blot analysis using anti-FLAG^{™} M2 antibody (Eastman Kodak, New Haven, CT). The scFv containing fractions were pooled, concentrated and purified to homogeneity by affinity chromatography using an anti-FLAG^{™} M2 antibody affinity resin (Brizzard *et al*, 1994). The affinity resin was equilibrated in PBS pH 7.4 and bound protein was eluted with 0.1 M glycine buffer, pH 3.0 and immediately neutralised with 1M Tris solution. Purified scFvs were concentrated to -1-2 mg/ml, dialysed against PBS, pH 7.4 which contained 0.02% (w/v) sodium azide and stored frozen.

The purity of the scFvs was monitored by SDS-PAGE and Western blot analysis as described previously (Kortt *et al*, 1994). The concentrations of the scFv fragments were determined spectrophotometrically using the values for the extinction coefficient (ε^{0.1%}) at 280 nm of 1.69 for scFv-15, 1.71 for scFv-10, 1.73 for scFv-5 and 1.75 for scFv-0 calculated from the protein sequence as described by Gill and von Hippel (1989).

For N-terminal sequence analysis of the intact scFv-0 and scFv-5 and the two lower molecular mass cleavage products, the protein bands obtained on SDS-PAGE were blotted on to a Selex 20 membrane (Schleicher and Schuell GmbH, Germany), excised and sequenced using an Applied Biosystems Model 470A gas-phase sequencer.

Soluble NC10 scFv-10, scFv-5 and scFv-0 fragments were each purified using a two step procedure involving gel filtration and affinity chromatography after extraction of the *E*. *coli* membrane fraction with 6 M guanidine hydrochloride, and dialysis to remove denaturant. The solubilised protein obtained was first chromatographed on Sephadex G-100 gel filtration to resolve three peaks (peaks 1-3, as shown in Figure 4) from a broad low-molecular mass peak. SDS-PAGE and Western blot analysis of fractions across peaks 1-3 showed the presence of scFv-0 in peaks 1 and 2 (fractions 19-30, as shown in Figure 5), with most of the scFv in peak 2. In contrast, in a previous report the expression of NC10 scFv-15 resulted in most of the scFv-15 being recovered from peak 3 as a monomer (Kortt *et al*, 1994). Affinity chromatography of peak 2 from Figure 4 on an anti-FLAG M2^{™} Sepharose column yielded essentially homogeneous scFv-0 preparations with a major protein band visible at -27 kDa by SDS-PAGE analysis (Figure 5); the decreasing size of the linker in the NC10 scFv-15, -10, -5 and -0 constructs is apparent from the mobility of the protein bands (Figure 6). ScFv-5 and scFv-0 also contained a small component of the protein as a doublet at -14 and -15 kDa (Figure 6), of which the 14 kDa band reacted with the anti-FLAG M2 antibody on Western blotting, consistent with proteolysis in the linker region between the V_{H} and V_{L}-FLAG domains.

Affinity chromatography of the Sephadex G-100 peak 1 from Figure 4 of NC10 scFv-10 and scFv-5 on an anti-FLAG^{™} M2 antibody column yielded scFv preparations which were aggregated; attempts to refold or dissociate the aggregates with ethylene glycol (Kortt *et al*, 1994) were unsuccessful. This material was not only aggregated, but was probably misfolded as it showed no binding activity to N9 neuraminidase or the anti-idiotype 3-2G12 Fab'. All subsequent analyses were performed on scFvs isolated from Sephadex G-100 peak 2.

### Example 3 Molecular Mass of NC10 scFvs

Gel filtration on a calibrated Superdex 75 column of affinity purified scFvs showed that the NC10 scFv-10 (Figure 7) and scFv-5 eluted with an apparent molecular mass of 52 kDa (Table 2), indicating that both these molecules are non-covalent dimers of the expressed 27 kDa NC10 scFv molecules. Although NC10 scFv-5 and NC10 scFv-10 yielded predominantly dimer, very small amounts of higher molecular mass components were observed, as shown in Figure 7 Panel b.

Gel filtration of affinity-purified NC10 scFv-0 yielded a single major symmetrical peak with an apparent molecular mass of approximately 70 kDa (Figure 7, Table 2). Since gel filtration behaviour depends on the size and shape of the molecule, the molecular mass of scFv-10, scFv-5, and scFv-0 was determined by sedimentation equilibrium as described above in order to obtain more accurate values.

A partial specific volume of 0.71ml/g was calculated for scFv-5 and scFv-0 from their amino acid compositions, and a partial specific volume of 0.7 ml/g was calculated for the scFv-neuraminidase complexes, from the amino acid compositions of scFvs and the amino acid and carbohydrate compositions of neuraminidase (Ward *et al*, 1983). A partial specific volume of 0.73 ml/g was assumed for the scFv-anti-idiotype 3-2G12 Fab' complex. The complexes for ultracentrifugation were prepared by size exclusion FPLC on Superose 6. The results are summarized in Table 2.

**Table 2**

| Molecular Mass of NC10 scFvs and of the Complexes Formed with Tern N9 Neuraminidase and Anti-Idiotype 3-2-GI2 Fab' Fragment | | | |
|---|---|---|---|
| | | MOLECULAR MASS | |
| | | Measured | Calculated |
| scFv-15 | monomer | 27,300 | 27,100 |
| | dimer | 54,300 | 54,200 |
| scFv-10 | dimer | 54,000 | 53,570 |
| scFv-5 | dimer | 52,440 | 52,940 |
| scFv-0 | trimer | 70,000* | 78,464 |
| | | 69,130 | |
| | | scFv-tern N9 neuraminidase complex | |
| | | Measured | Calculated |
| scFv-15 | monomer | 298,000 | 298,400 |
| | dimer | 610,000 | 596,800 |
| scFv-10 | dimer | 596,000 | 594,280 |
| scFv-5 | dimer | 595,000 | 591,760 |
| | | scFv-anti-idiotype 3-2-G12 Fab' complex | |
| | | Measured | Calculated |
| scFv-15 | monomer | 77,900 | 77,100 |
| scFv-10 | dimer | nd | |
| scFv-5 | dimer | 156,000 | 152,940 |
| scFv-0 | trimer | 212,000# | 220,000 |

Molecular mass determined in 0.05M phosphate, 0.15 M NaCl, pH 7.4 by sedimentation equilibrium analysis in a Beckman model XLA ultracentrifuge.

# Apparent average molecular mass obtained by fitting data in Figure 9, assuming a single species.

* Molecular mass estimated by gel filtration on Superdex 75 in 0.05 M phosphate, 0.15 M NaCl, pH 7.4 at a flow rate of 0.5 ml/min at 20°C. The molecular masses of the complexes were calculated using a Mᵣ of 50,000 for the Fab' and 190,000 for tern N9 neuraminidase.

The molecular masses of 54 and 52.4 kDa, respectively, for scFv-10 and scFv-5 confirmed that they were dimers. The molecular mass of 69 kDa determined for the NC10 scFv-0 suggested that it was a trimer composed of three scFv-0 chains, but this molecular mass is lower than expected for such a trimer (calculated Mᵣ of 78 kDa). Analysis of the sedimentation data gave linear In c versus r² plots (Van Holde, 1975), indicating that under the conditions of the experiment scFv-5 dimer and scFv-0 trimer showed no dissociation. Furthermore, the sedimentation equilibrium results did not indicate a rapid equilibrium between dimer and trimer species to account for this apparently low molecular mass for NC10 scFv-0 trimer.

Purified NC10 scFv-5 and scFv-10 dimers at concentrations of ∼1mg/ml showed no propensity to form higher molecular mass aggregates at 4°C, but on freezing and thawing higher-molecular mass multimers were formed (data not shown). These multimers were dissociated readily in the presence of 60% ethylene glycol, which suppresses hydrophobic interactions. In contrast the NC10 scFv-0 showed no propensity to aggregate on freezing and thawing, even at relatively high protein concentrations.

N-terminal analysis of the two bands from the Fv fragment produced during the isolation of the NC10 scFv-0 (Figure 6) also confirmed that the 15 kDa band was the V_{H} domain and that the 14 kDa band had the N-terminal sequence of **V S D I E L T Q T T**, indicating that a small amount of proteolysis had occurred at the penultimate bond (T-V) in the C-terminal sequence of the V_{H} domain (Figure 3).

### Example 4 Complexes formed between NC10 scFv dimers and trimers and tern N9 neuraminidase and anti-idiotype 3-2G12 Fab'

Influenza virus neuraminidase, a surface glycoprotein, is a tetrameric protein composed of four identical subunits attached via a polypeptide stalk to a lipid and matrix protein shell on the viral surface (Colman, 1989). Intact and active neuraminidase heads (Mᵣ 190 kDa) are released from the viral surface by proteolytic cleavage in the stalk region (Laver, 1978). The four subunits in the neuraminidase tetramer are arranged such that the enzyme active site and the epitope recognised by NC10 antibody are all located on the upper surface of the molecule (distal from the viral surface). This structural topology permits the binding in the same plane of four NC10 scFv-15 monomers or four Fab fragments (Colman et *al,* 1987; Tulip *et al,* 1992) such that the tetrameric complex resembles a flattened box or inverted table with the neuraminidase as the top and the four Fab fragments projecting as the legs from the plane at an angle of 45°. This suggests that a bivalent molecule may be able to cross-link two neuraminidase tetramers to form a 'sandwich' type complex (Figure 8a; Tulloch *et al,* 1989).

Size-exclusion FPLC on a calibrated Superose 6 column showed that both the NC10 scFv-10 (Figure 7) and NC10 scFv-5 dimers formed stable complexes with soluble neuraminidase with apparent molecular masses of approximately 600 kDa. The more accurate molecular mass determined by sedimentation equilibrium analysis for the scFv-10 and scFv-5-neuraminidase complexes was 596 kDa (Table 2). This complex Mᵣ is consistent with four scFv dimers (each 52 kDa) cross-linking two neuraminidase molecules (each 190 kDa) in a 'sandwich' complex, as illustrated schematically in Figure 8a, and demonstrates that the scFv-10 and scFv-5 dimers are bivalent.

Gel filtration of the isolated 600 kDa NC10 scFv-10-neuraminidase complex showed that it was extremely stable to dilution, with only a small amount of free neuraminidase and NC10 scFv-10 appearing when complex at a concentration of 2 nM was run on the Superose 6 column. The linearity of the In c versus r² plots (Van Holde, 1975) of the sedimentation data, demonstrated in Example 3, showed that both complexes were homogeneous with respect to molecular mass and indicated that discrete and stoichiometric complexes were formed. Complex formation with different molecular ratios of scFv to neuraminidase (from 1:4 to 8:1) yielded only the 600 kDa complex. Interestingly, complexes with 4 scFv dimers binding to 1 neuraminidase (-400 kDa) or aggregated complexes in which more than two neuraminidases were cross-linked were not observed.

Size exclusion FPLC on Superose 6 showed that anti-idiotype 3-2G12 Fab' formed stable complexes with NC10 scFv-15 monomer, NC10 scFv-5 and NC10 scFv-0. Sedimentation equilibrium analyses of the isolated complexes gave molecular masses consistent with the scFv-15 binding one Fab', NC10 scFv-5 binding two Fab's and the NC10 scFv-0 binding three Fab' molecules, as shown in Table 2 and Figure 9. The linearity of the 1n c versus r² plots of the sedimentation data (Figure 9) showed that the complexes with NC10 scFv-15 monomer and NC10 scFv-5 dimer were homogeneous, and that discrete and stoichiometric complexes were formed. The equilibrium data for the complex with NC10 scFv-0 showed a very slight curvature on linear transformation (Figure 9). The fit to the data yielded an average Mᵣ of 212,000, which corresponds closely to the expected Mᵣ for a complex of three Fab' binding per NC10 scFv-0 (Table 2). The slight curvature of the transformed data may indicate a small degree of dissociation of the complex under the experimental conditions. The result with the NC10 scFv-5 confirmed that the dimer is bivalent,as illustrated in Figure 8b, and that the NC10 scFv-0 with no linker is a trimer with three active antigen binding sites, as illustrated schematically in Figures 8c and 8d.

It will be appreciated that Figure 8 represents a schematic representation of the complexes, and that there is considerable flexibility in the linker region joining the scFvs, which cannot be depicted. Note, however, that the boomerang-shaped structure (Figure 8b), rather than a linear structure, can readily accommodate the 45° angle of projection of the scFv from the plane of the neuraminidase required for four dimers to cross-link simultaneously two neuraminidase molecules in the 'sandwich' complex as indicated in Figure 8a. Similar flexibility of a different scFv-5 dimer has recently been modelled (Holliger et *al,* 1996), but has hitherto not been demonstrated experimentally.

Electron micrographs of the NC10 scFv-5 diabodies complexed with two anti-idiotype 3-2G12 Fab molecules (Mᵣ ∼156 kDa) showed boomerang-shaped projections with the angle between the two arms ranging from about 60°-180°, as shown in Figure 16. The mean angle was 122°, with an approximately normal distribution of angles about the mean (Table 3). Each arm corresponds to an Fab molecule (Figures 1 and 8b), and, despite the potential 'elbow' flexibility between Fv and C modules, appears as a relatively rigid, linear molecular rod which extends outwards from the antigen binding sites. Linearity of the Fab arms under the current imaging conditions was confirmed by the appearance of free 3-2G12 anti-idiotype Fabs imaged in conjunction with triabodies. The variation in the angle between the arms indicates that there is considerable flexibility in the linker region joining the two scFvs in the diabody. Measurements of the arm lengths are summarized in Table 3.

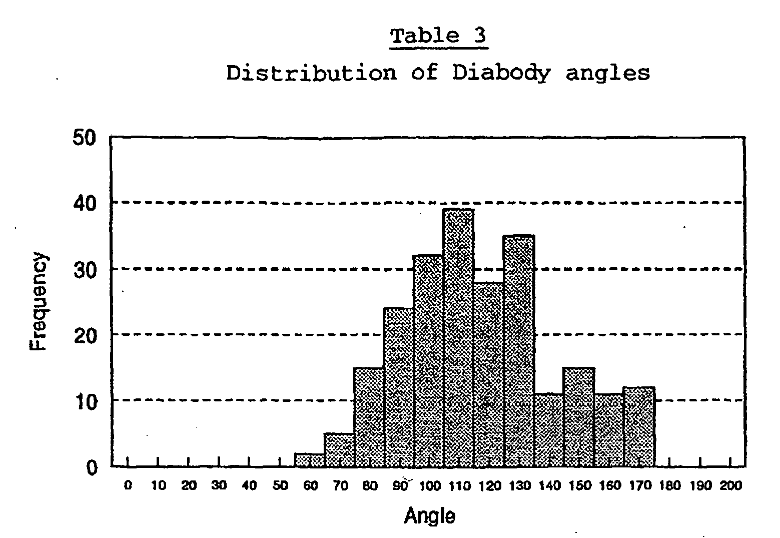

### Diabody Measurements

| | Mean length (arbitrary units) | Standard deviation |
|---|---|---|
| end-to-end | 47.0 | 4.8 |
| shorter arm | 21.6 | 2.9 |
| longer arm | 25.4 | 2.6 |
| Mean angle | 122.4° | |
| Min angle | 60.5° | |
| Max angle | 178.8° | |

In micrographs of NC10 scFv-0 triabodies complexed with three 3-2G12 Fab molecules (Mᵣ ∼212 kDa), most fields showed a mixture of predominantly Y-shaped and V-shaped projections (Figure 16a). There was some variation in particle appearance depending on the thickness of the stain on the carbon film. The Y-shaped projections were interpreted as tripods (viewed from above), which had adopted an orientation in which all three legs (ie the distal ends of the three Fab molecules) were in contact with the carbon film. The three Fab legs were separated by two angles of mean 136° and one of mean 80°. However, the range of angles was such that for approximately 10% of particles the arms were evenly spaced, with angles all 120° (+/- 5°).

The Y-shaped projections were unlikely to be planar, as invariably one of the Fab legs appeared foreshortened. The V-shaped projections were interpreted as tripods (triabody complexes) lying on their sides on the carbon film, with two Fab legs forming the V and the third Fab leg extending upward and out of the stain, which would account for the increase in density sometimes observed at the junction of the V.

The V-shaped structures were clearly different to the boomerang-shaped diabody complexes, both in the angle between Fab arms and in the projected density in the centre of the molecules, consistent with the expected models (Figure 1). The interpretation of tripods lying on their side is consistent with the appearance of a few projections with all 3 Fab legs pointing in the same direction.

Triabodies are obviously flexible molecules, with observed angles between Fab arms in the NC10 triabody/Fab complexes distributed around two angles of mean 136° and one of mean 80°, and are not rigid molecules as shown schematically in Figure 1.

### Example 5 Binding interactions of NC10 scFvs measured on the BIAcore^{™}

### a) Binding of NC10 scFvs to anti-idiotype 3-2G12 Fab'

In a series of experiments anti-idiotype 3-2G12 Fab' and the NC10 scFv-15 monomer, scFv-10, scFv-5 and scFv-0 were also immobilised at pH 4.0 via their amine groups. Binding analyses were performed in HBS buffer (10 mM HEPES, 0.15 M NaCl, 3.4 mM EDTA, 0.005% surfactant P20, pH 7.4) at a constant flow rate of 5 µl/min.

Immobilised 3-2G12 Fab' could be regenerated with 10 µl 0.01 M sodium acetate buffer, pH 3.0 without loss of binding activity. A comparison of the binding of the NC10 scFv-15 monomer, scFv-10 and scFv-5 dimers, and scFv-0 trimer showed that the monomer dissociated rapidly, and non-linear least squares analysis of the dissociation and association phase, using the single exponential form of the rate equation, gave a good fit to the experimental data. These results are shown in Figure 10, and the rate constants determined are given in Table 4.

**Table 4**

| Immobilised | Analyte | apparent Kₐ | apparent k_{d} | apparent Kₐ |
|---|---|---|---|---|
| ligand | | (M⁻¹ s⁻¹) | (s⁻¹) | (M⁻¹) |
| neuraminidase | scFv-15 | 2.6±0.3x10⁵ | 5.2±0.3x10⁻³ | 5.0±0.9x10⁷ |
| | monomer | | | |
| 3-2-G12 Fab' | scFv-15 | 7.4±0.6x10⁵ | 1.74±0.06x10⁻³ | 4.2±0.5x10⁸ |
| | monomer | | | |
| scFv-15 | 3-2-G12 Fab' | 5±1x10⁵ | 2,1±0.1x10⁻³ | 2.5±0.63x10⁸ |
| monomer | | | | |
| scFv-10 | 3-2-G12 Fab' | 3.7±0.4x10⁵ | 2.9±0.2x10⁻³ | 1.3±0.23x10⁸ |
| dimer | | | | |
| scFv-5 | 3-2-G12 Fab' | 3.5±0.9x10⁵ | 3.3±0.1x10⁻³ | 1.06±0.3x10⁸ |
| dimer | | | | |
| scFv-0 | 3-2-G12 Fab' | 2.6±0.1x10⁵ | 2.3±0.1x10⁻³ | 1.13±0.9x10⁸ |
| trimer | | | | |

This table shows the apparent kinetic constants for the binding of NC10 scFv-15 monomer to immobilised tern N9 neuraminidase and anti-idiotype 3-2-G12 Fab' fragment determined in the BIAcore^{™} The kinetic constants were evaluated from the association and dissociation phase using non-linear fitting procedures described in BIAevaluation 2.1. The binding experiments were performed in 10 mM HEPES, 0.15 NaCl,3.4 mM EDTA, 0.005% surfactant P20, pH 7.4 at a flow rate of 5 µl/min. Tern N9 neuraminidase (1360 RU)and 3-2-G12 Fab' (1000 RU)were immobilised via amine groups using the standard NHS/EDC coupling procedure.

The NC10 scFv-10 and scFv-5 dimers and scFv-0 trimer/anti-idiotype complexes showed apparently slower dissociation, as illustrated in Figure 10, consistent with multivalent binding, and kinetic analysis was not performed because this effect invalidates the 1:1 interaction model used to evaluate BIAcore^{™} data. To resolve this problem the interaction format was inverted by immobilisation of each NC10 scFv and using the anti-idiotype Fab' as the analyte. NC10 scFv-15 monomer (2000 RU) and NC10 scFv-1-dimer (200 RU), scFv-5 dimer (200 RU) and scFv-0 trimer (450 RU) were also immobilised via amine groups, using the standard NHS/EDC coupling procedure. This orientation of the reagents achieves experimentally the 1:1 interaction model required to determine the rate constants. The kinetic binding constants for the binding of 3-2G12 Fab to immobilised NC10 scFv-15 monomer, NC10 scFv-10 dimer, NC10 scFv-5 dimer and the NC10 scFv-0 trimer are given in Table 4, and the properties of the immobilised NC10 scFvs in the BIAcore^{™} are presented in sections b i) and ii) below.

### b) Binding of anti-idiotype 3-2G12 Fab' to immobilised NC10 scFv-15 monomer and scFv-10, scFv-5 and scFv-0

### i) NC10 scFv-15 monomer

Although the scFv-15 monomer was readily immobilised (- 2000 Response Units; RU), less than 10% of the protein was active, as indicated by the total amount of anti-idiotype Fab' that could be bound to the surface as calculated from the RU increase. Logarithmic transformation of the dissociation phase data showed significant deviation from linearity which permitted only approximate values of the binding constants to be estimated (Table 4).

### ii) scFv-10, scFv-5 and scFv-0

In contrast, the three NC10 scFvs with the shorter linkers were not readily immobilised via their amine groups, since only 200-550 RU of protein could be immobilised after several injections of protein at a flow rate of 2 µl/min. Binding experiments with anti-idiotype 3-2G12 Fab' indicated that approximately 30-50% of the immobilised scFv-10, scFv-5 and scFv-0 were active, as calculated from the total bound RU response. The results are shown in Table 4. As for immobilised NC10 scFv-15 monomer, analysis of the data showed deviation from linearity on logarithmic transformation of dissociation data and poor fits when the data was analysed by non-linear regression. These non-ideal effects associated with BIAcore^{™} binding data may arise either from the rate of molecular diffusion to the surface contributing to the kinetic constants (mass transfer effect) (Glaser, 1993; Karlsson *et al,* 1994) or from the binding heterogeneity of the immobilised molecules resulting from the non-specific immobilisation procedure used, or both. These effects contribute to lowering the measured rate constants and affect the estimated binding constants. A comparison of the rate constants for the binding of 3-2G12 Fab to each of the four immobilised NC10 scFvs shows that the apparent affinity for the interaction of 3-2G12 Fab with each scFv is similar, as shown in Table 4. Increases in affinity that are shown in Figure 10 for dimeric and trimeric scFvs binding to immobilised 3-2G12 Fab therefore arise from multivalent binding (an avidity effect) when dimers or trimers are used as analytes in either BIAcore biosensor or ELISA affinity measurements.

### Example 6 Construction, Expression and Activity of NC10 scFv with 1, 2, 3 and 4 Residue Linkers

The starting template for construction of the short linkered scFvs was the zero-linked NC10 scFv-0 gene construct in the vector pPOW as described in Example 1, in which the 5' end of the V_{L} sequence is linked directly to the 3' end of the V_{H} sequence. The constructions were designed to add nucleotides coding for one, two, three or four glycine residues between the 3' end of the V_{H} and the 5' end of the V_{L} sequence.

Four sets of complementary oligonucleotide primers were synthesised as shown in Table 5 to add the extra codons between the V_{H} and V_{L} sequences, using the QuikChange^{™} Site-Directed Mutagenesis procedure (Stratagene Cloning Systems, La Jolla, CA).

**Table 5**

| DNA sequences of Synthetic Oligonucleotides used to insert codons between V_{H} and V_{L} domains of NC10 scFv-0 to create NC10 scFv-1, scFv-2, scFv-3, scFv-4 using QuickChange^{®} Mutagenesis. Additional glycine codons shown in lowercase. | | | |
|---|---|---|---|
| Construct | Complementary Oligonucleotide Pair | | SEQ ID NO. |
| scFv-1 | 5' GGG ACC ACG GTC ACC GTC TCC ggt GAT ATC GAG CTC ACA CAG 3' | | 9 |
| | 3' CCC TGG TGC CAG TGG CAG AGG cca CTA TAG CTC GAG TGT GTC 5' | | 10 |
| scFv-2 | 5' GGG ACC ACG GTC ACC GTC TCC ggt ggt GAT ATC GAG CTC ACA CAG 3' | | 11 |
| | 3' CCC TGG TGC CAG TGG CAG AGG cca cca CTA TAG CTC GAG TGT GTC 5' | | 12 |
| scFv-3 | 5' GGG ACC ACG GTC ACC GTC ICC ggt ggt ggt GAT ATC GAG CTC ACA CAG 5' | | 13 |
| | 3' CCC TGG TGC CAG TGG CAG AGG cca cca cca CTA TAG CTC GAG TGT GTC 3' | | 14 |
| scFv-4 | 5' GGG ACC ACG GTC ACC GTC TCC ggt ggt ggt ggt GAT ATC GAG CTC ACA CAG 3' | | 15 |
| | 3' CCC TGG TGC CAG TGG CAG AGG cca cca cca cca CTA TAG CTC GAG TGT GTC 5' | | 16 |

15 ng NC10 scFv-0 DNA was subjected to PCR in a 50 µl reaction volume containing 5µl reaction buffer supplied with the kit, 20 pmoles of the complementary oligonucleotide primers, 2.5 nmoles of each dNTP, and 2.5 units *Pfu* DNA polymerase. Thermal cycling conditions were: (95°C, 30 secs) 1 cycle; (95°C, 30 sec; 55°C, 1 min; 68 °C , 12 min) 18 cycles. 1 µl *Dpn I* restriction enzyme (10 U/µl) was added to each sample and incubated at 37°C for 90 min to digest dam methylated, non-mutated parental DNA. 2 µl of each reaction mixture was used to transform electrocompetent XL1-Blue cells (*recA endA 1 gyrA96 thi-1 hsdR17 supE44 relA1 lac [f' proAB 1acI^{q}Z*Δ*M15 Tn10 (tetr)])* (1 x 10⁹ cfu/µg), aliquots of which were incubated overnight on YT-amp₁₀₀ plates at 30°C.

Mutants containing the correct nucleotide insertions were selected by DNA sequencing of plasmid DNA from a number of individual colonies across the region targeted for mutation, using Sequenase ver 2.0 (US Biochemicals) and the oligonucleotide primer TACATGCAGCTCAGCAGCCTGAC (SEQ ID NO. 17). Clones having the correct mutations were subjected to small scale expression in 5 ml 2YT/amp₂₀₀ as described in Malby *et al* (1993) to confirm that the construct could produce a full length, inframe product. Culture samples were analysed by SDS-PAGE and Western Blot with anti-FLAG^{®} M2 antibody. The selection criterion was a positive reaction at the correct migration positions. One positive clone was selected from this screen for each of the four constructions.

Large-scale expression and purification of NC10 scFv-1, scFv-2, scFv-3 and scFv-4 were performed as described in Example 2, but with the chromatography step on Sephadex G-100 omitted. SDS PAGE and Western Blot of the bound fraction from affinity chromatography on immobilised anti FLAG revealed that they contained predominantly NC10 scFv.

### Estimation of Molecular Mass of NC10 scFv-1, scFv-2, sCFv-3 and scFv-4

Aliquots of affinity purified NC10 scFv-1, scFv-2, scFv-3, scFv-4 were individually analysed by FPLC on a calibrated Superose 12 column. Elution profiles are shown in Figure 11. NC10 scFv-1 and scFv-2 yielded a major peak eluting in the position of a trimer, similar to that described for scFv-0. The position of the major eluting peak for scFv-3 and scFv-4 was the same as that observed for a dimer, as seen for scFv-5. These results indicate that the extension of the linker from 2 to 3 glycine residues between the V_{H} and V_{L} domains of NC10 is sufficient to allow the preferred multimerisation state of the scFv to change from trimer (as is seen with scFv-0) to dimer (as is seen with scFv-5).

### Activity of TBRs - Formation of complexes with 3-2G12 Fab' and EM imaging.

Complexes were formed between 3-2-G12 Fab' and affinity purified NC10 scFv-2 and scFv-3, as described for scFv-0 and scFv-5 (Example 4), isolated by FPLC on Superose 6 and used for EM imaging, also as described for scFv-0 and scFv-5.

The absence of any free scFv peak in the FPLC profile after the formation of complexes in the presence of excess Fab' indicated that both scFv-2 and scFv-3 were completely active. The elution time for the scFv-2/Fab complex was identical to that found previously for the scFv-0/Fab complex, and is consistent with scFv-2 being a trimer. The scFv-3/Fab complex had an identical elution time to that found previously for the scFv-5/Fab complex, and is consistent with the scFv-3 being a dimer.

EM images of scFv-2/Fab and scFv-3/Fab complexes showed results which were consistent with our previous observations that the NC10 scFv-2 was a stable trimer similar to scFv-0 and scFv-3 was a stable dimer similar to scFv-5. These images appear identical to either scFv-5 dimer complexes or scFv-0 trimer complexes shown in Figure 16).

### Example 7 Construction and synthesis of 11-1G10 scFv-0

The V_{H} and V_{L} genes were amplified by PCR from the parent 11-1G10 hybridoma, and joined into an scFv-0 gene by ligation between codons for C-terminal V_{H}-Ser¹¹³ and N-terminal V_{L}-Gln¹ by PCR overlap-extension. For 11-1G10 the zero-linkered scFv is defined as the direct linkage of V_{H}-Ser¹¹³ to V_{L}-Gln¹. The scFv-0 gene was cloned into the *Sfi*1*-Not*1 sites of the expression vector pGC which provides an N-terminal pelB leader sequence and C-terminal FLAG octapeptide tag tail (Coia *et al,* 1996). The entire DNA sequence of the cloned scFv-0 insert was determined using DNA purified by alkaline lysis and sequencing reactions performed using the PRISM Cycle Sequencing Kit (ABI). This confirmed that the 11-1G10 scFv-0 gene comprised a direct ligation between codons for the C-terminal V_{H}-Ser¹¹³ and N-terminal V_{L}-Gln¹.

HB101 E. coli containing the scFv-0 gene in pGC were grown in 2 x YT supplemented with 100 µg/ml ampicillin and 1% glucose at 37°C overnight and then subcultured in the absence of glucose at an A₆₀₀ of 0.1, and grown at 21°C until A₆₀₀ was 1.0. Expression was induced by addition of IPTG to 1mM and cells cultured for 16 hours at 21°C under conditions which release the contents of the periplasmic space into the culture supernatant, presumably by cell lysis, to yield soluble and biologically active scFv (Coia *et al,* 1996). Cells and culture supernatant were separated by centrifugation, and samples of cell pellet and supernatant were analysed on a 15% SDS-PAGE gel, followed by Western blot analysis using M2 anti-FLAG antibody (Kortt *et al,* 1994) and goat anti-mouse IgG (H+L)^{HRP} (BioRad) as the second antibody to visualise the expressed product.

The expressed scFv-0 was purified from supernatant by precipitation with ammonium sulphate to 70% saturation at 21°C followed by centrifugation at 10000g for 15 minutes. The aqueous phase was discarded, and the pellet resuspended and dialysed in PBS at 4°C overnight. Insoluble material was removed by centrifugation at 70,000g and the supernatant was filtered through a 0.22 µm membrane and affinity purified on either an M2 anti-FLAG antibody affinity column (Brizzard *et al*, 1994) or an NC41 Fab Sepharose 4B affinity column. The affinity resin was equilibrated in TBS (0.025M Tris-buffered saline, pH 7.4) and bound protein was eluted with gentle elution buffer (Pierce). The scFv-0 was concentrated to about 1 mg/ml, dialysed against TBS and stored at 4°C. SDS-PAGE analysis of the affinity purified scFv-0 revealed a single protein band of 27 kDa which on Western analysis reacted with the anti-FLAG M2 antibody (Figure 12). N-terminal sequence analysis of the 27 kDa protein gave the expected sequence for the N-terminus of the 11-1G10 V_{H} domain, and confirmed that the pelB leader sequence had been correctly cleaved.

### Example 8 Size Exclusion FPLC Chromatography, Molecular Mass Determination and Binding Analysis of 11-1G10 scFv Fragments

The affinity-purified 11-1G10 scFv-0 was as described in Example 5. For the other proteins described in this example, the 11-1G10 scFv-15 (comprising a 15 residue linker in the orientation V_{H}-(Gly₄Ser)₃-V_{L}) was synthesised under similar conditions to the scFv-0 described in Example 5 above. The 11-1G10 scFv-15 was isolated by gel filtration as a 27kDa monomer and shown to be stable at 4°C for several weeks, similar to previous studies with different scFv-15 fragments. NC41 and 11-1G10 Fab fragments were prepared by proteolysis from the parent hybridoma IgG as described previously in this specification. 11-1G10 scFv-0 and scFv-15 were fractionated by size exclusion FPLC on either a Superdex 75 HR10/30 column or a Superose 12 HR10/30 column (Pharmacia) in PBS to determine the molecular size and aggregation state.

The complexes formed between I1-1G10 scFv and NC41 Fab were analysed and isolated by size exclusion FPLC on a Superose 12 column in PBS (flow rate 0.5 ml/min). The FPLC columns were calibrated with standard proteins as described (Kortt *et al,* 1994). The molecular mass of each isolated complex was determined by sedimentation equilibrium on a Beckman model XLA centrifuge as described previously (Kortt *et al,* 1994) using partial specific volumes calculated from amino acid compositions. An upgraded Pharmacia BIAcore^{™} 1000 was used for analysis of the binding of monomeric 11-1G10 scFv-15 and trimeric 11-1G10 scFv-0 to immobilised NC41 Fab as described (Kortt *et al,* 1994). The resulting binding curves were analysed with BIAevaluation 2.1 software (Pharmacia Biosensor), to obtain values for the apparent dissociation rate constants.

Gel filtration of affinity purified scFv-0 by FPLC on either a Superdex 75 column (Figure 13) or a Superose 12 column (Figure 14) revealed a single peak of Mᵣ ∼85kDa consistent with the calculated molecular mass of a trimer (calculated Mᵣ 79.4kDa). Gel filtration of the scFv-0 preparation showed no evidence of monomers and dimers, and no evidence of proteolytic degradation to single V-domains. Sedimentation equilibrium analysis indicated that the scFv-0 migrated as a distinct species with Mᵣ ∼85kDa (Table 6), consistent with a trimeric conformation, and there was no evidence for a dimeric species which might exist in rapid equilibrium with the trimer species.

**Table 6**

| Sedimentation equilibrium data for complexes of 11-1G10 scFv-15 monomer and scFv-0 trimer with NC41 Fab | | |
|---|---|---|
| Sample | Calculated | Experimental |
| Monomer + NC41 Fab | 75700 | 78600 |
| 28427 + 47273 | | |
| Trimer | 79398 | 85000 |
| Trimer + NC41 Fab | 221217 | 262000 |
| 79398 + 141819 | | |

The complexes of NC41 Fab with either scFv-15 monomer or scFv-0 trimer were isolated by size exclusion FPLC chromatography and analysed by sedimentation equilibrium in a Beckman Model XLA ultracentrifuge. The molecular mass was determined experimentally by the method described by Kortt *et al,*1994 at 20°C. The calculated MW of NC41 Fab is 47273 Da, scFv-15 is 28427 Da and scFv-0 is 26466 Da.

In comparison, the scFv-15 fragment of 11-1G10 (comprising a 15 residue linker in the orientation V_{H}-(Gly₄Ser)₃-V_{L} ) was also synthesised using the pGC vector in HB2151 *E.coli* cells, and then purified as a stable monomer with a Mᵣ ∼27 kDa determined by gel filtration and sedimentation equilibrium (Figure 13). Previous examples have shown gel filtration and sedimentation equilibrium studies of NC10 scFv fragments that revealed that scFv-15 monomers possessed an Mᵣ ∼27 kDa, scFv-5 dimers Mᵣ ∼54kDa and scFv-0 trimers Mᵣ ∼70kDa. Thus, the calculated and experimental Mᵣ of ∼27kDa for monomeric scFv-15 derived from both 11-1G10 and NC10 antibodies were almost identical, whereas scFv-0 from 11-1G10 exhibited a Mᵣ ∼85kDa slightly larger than that predicted for a trimer (79 kDa) and scFv-0 from NC10 a Mᵣ ∼70 kDa slightly smaller than a trimer.

Gel filtration analysis by FPLC on a Superose 12 column showed that all the scFv-0 interacted with NC41 Fab to form a stable complex of Mᵣ -245 kDa (Figure 14), whilst scFv-15 monomer interacted with NC41 Fab to form a stable complex of Mᵣ -79kDa (not shown). The molecular masses of these complexes were determined by sedimentation equilibrium analysis to be 262 kDa and 78.6 kDa respectively (Table 6). Furthermore, both isolated complexes were stable to dilution and freezing (data not shown). These data are consistent with the trimeric scFv-0 binding three Fab molecules whilst the monomeric scFv-15 formed a 1:1 complex with Fab. Comparison of the binding of scFv-15 monomer and scFv-0 trimer to immobilised NC41 Fab by BIAcore^{™} (Figure 15) showed that the apparent dissociation rate of the scFv-0 trimer/NC41 Fab complex (k_{d} ∼ 8.2 x 10⁻⁵ s⁻¹) was approximately 4-fold slower than that for the scFv-15 monomer/NC41 Fab complex (k_{d} ∼ 3.2 x 10⁻⁴. s⁻¹). The 4-fold reduced apparent dissociation rate for the 11-1G10 scFv-0 trimer is similar to earlier Example 5 for the NC10 scFv-0 trimer, and can be attributed to multivalent binding which results in the increased functional affinity for both scFv-0 trimers.

### Example 9 Design and Synthesis of NC10 scFv-0 with a (V_{L}-V_{H}) Orientation, and Size Exclusion FPLC Chromatography

The NC10 scFv-0 (V_{L}-V_{H}) gene encoded the pelB leader immediately followed by the N-terminal residues of DIEL for the V_{L} gene. The C-terminus of the V_{L} gene encoded residues KLEIR¹⁰⁷ (where R is unusual for V_{L}). The N terminus of the V_{H} (residues QVQL) immediately followed to form a linkerless construct. The C-terminus of the V_{H} terminated with residues VTS¹¹², and was immediately followed by a C-terminal FLAG^{™} sequence for affinity purification. The NC10 scFv-0 V_{L}-V_{H} gene was, then subcloned and expressed in the heat inducible expression vector pPOW using methods described in Kortt *et al,* 1994 and Examples 1-4 above. The isolation of NC10 scFv-0 (V_{L}-V_{H} ) from the *E. coli* cell pellet required extraction and solubilisation with 6M GuHCl, preliminary purification using a Sephadex G-100 column, and affinity purification using an anti-FLAG M2 affinity column, using methods described in Kortt et *al,* 1994.

SDS-PAGE and Western blot analysis of purified NC10 scFv-0 (V_{L}-V_{H}) gave a major protein band at -30 kDa. FPLC analysis of purified scFv-(V_{L}-V_{H}) on a Superose 12 HR10/30 column (Pharmacia) run at a flow rate of 0.5 ml/min gave a major protein peak eluting at 22.01 minutes with a distinct shoulder on the trailing edge of the peak (Figure 17). The NC10 scFv-0 (V_{L}-V_{H}) trimer eluted at 23.19 minutes on this column. FPLC analysis on two Superose 12 HR10/30 columns linked in tandem separated two protein peaks from the affinity-purified NC10 scFv-0 (V_{L}-V_{H}), with apparent molecular masses of 108 kDa and 78 kDa. On SDS-PAGE and Western blot analysis both these peaks yielded a band at -30 kDa. The FPLC analysis using the two Superose columns demonstrated that NC10 scFv-0 (V_{L}-V_{H}) forms both trimers (Mᵣ -78 kDa) and tetramers (108 kDa) which are stable and can be isolated on gel filtration.

Purified NC10 scFv-0 (V_{L}-V_{H}) tetramer and NC10 scFv-0 (V_{L}-V_{H} ) trimer reacted with anti-idiotype 3-2G12v Fab to yield complexes of 4 Fab/tetramer and 3 Fab/trimer, demonstrating the tetravalent and trivalent nature of the two NC10 scFv-0 (V_{L}-V_{H}) molecules. EM analysis of complexes of the isolated NC10 scFv-0 V_{L}-V_{H} trimer and tetramer complexed with 3-2G12 anti-idiotype Fab showed images of tripods and crosses consistent with the trimers having 3 functional TBRs and the tetramers having 4 active TBRs, as shown in Figure 16c and d.

### Example 10 Design and synthesis of C215 scFv-0

The strategy for construction of the zero-linked C215 scFv antibody gene construct was as described in Example 7 in which the 5' end of the V_{L} sequence (Glu¹) is linked directly to the 3' end of the V_{H} sequence (Ser¹¹³). The V_{H} and V_{L} genes of C215 (Forsberg *et al,* 1997)were amplified by PCR from the parent Fab coding region, and joined into an scFv-0 gene by PCR overlap-extension. The scFv-0 gene was cloned into the *Sfi*1-*Not*1 sites of the expression vector pGC, which provides an N-terminal pelB leader sequence and C-terminal FLAG octapeptide tag tail (Coia *et al,* 1996). The C-terminus of the V_{L} terminated with residues ELK¹⁰⁷, and was immediately followed by the C-terminal FLAG^{™} sequence for affinity purification. The scFv-0-linker gene was also cloned into the NdeI-EcoRI sites of the expression vector pRSET^{™}, which is a cytoplasmic expression vector. The oligonucleotides used to amplify the C215 with the correct restriction sites for cloning into pRSET are:
FORWARD: GATATACATATGCAGGTCCAACTGCAGCAG (SEQ ID NO. 18)
BACKWARD: ATTAGGCGGGCTGAATTCTTATTTATCATC (SEQ ID NO. 19)

The entire DNA sequences of the cloned scFv-0 inserts were determined using DNA purified by alkaline lysis and sequencing reactions were performed using the PRISM Cycle Sequencing Kit (ABI). This confirmed that the C215 scFv-0 gene comprised a direct ligation between codons for the C-terminal V_{H}-Ser¹²¹ and N-terminal V_{L}-Glu¹.

HB101 *E. coli* expression of the C215 scFv-0 was performed as detailed in Example 7 The C215 scFv-0 was concentrated to about 1 mg/ml, dialysed against TBS and stored at 4°C. SDS-PAGE analysis of the affinity purified scFv-0 revealed a single protein band of Mᵣ ∼28 kDa which on Western analysis reacted with the anti-FLAG M2 antibody. N-terminal sequence analysis of the Mᵣ -28 kDa, protein gave the expected sequence for the N-terminus of the C215 V_{H} domain, and confirmed that the pelB leader sequence had been correctly cleaved.

### Example 11 Size exclusion FPLC chromatography of C215 scFv-0

The affinity-purified C215 scFv-0 was as described in Example 10.

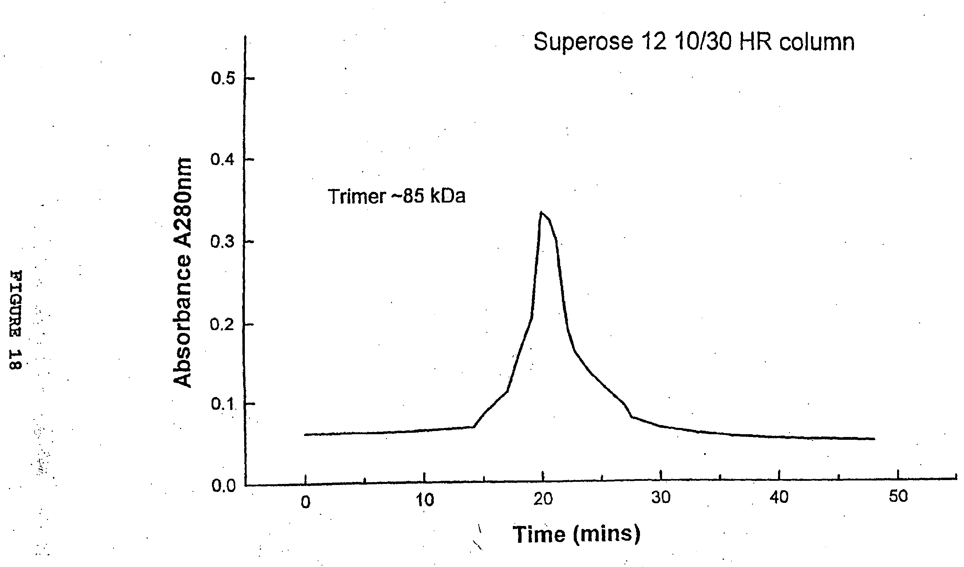

Gel filtration of affinity-purified C215 scFv-0 by FPLC on a calibrated Superose 12 column (HR10/30) revealed a major peak of Mᵣ ∼85kDa, (an apparent trimer) with a retention time of 20.20 mins.as shown in Figure 18. SDS PAGE of the scFv-0 preparation showed no evidence of proteolytic degradation to single V-domains. C215 scFv-5 ran as a dimer (not shown).

### Example 12 Design and construction of trispecfic triabody of Ig-like V domains

### Construction of three discrete bispecific Ig-like V domains which are designed to assemble into trimers with three different binding specificities: CTLA-4-0 linked to CD86, CTLA-4-0 linked to UV-3 VL and UV-3 VH-0 linked to CD86.

The Ig-like V domains were separately amplified by PCR from the parent coding region with appropriate oligonucleotides pairs which are listed in table 6: #4474/#4475(UV-3 VH), #4480/4481 (UV-3 VL), #4470/#4471 (human CTLA-4) (Dariavach 1988), #4472/#4473 (CD86 V domain) respectively.

**Table 7**

| DNA Sequences of Oligonucleotides Used in the Amplification of Ig-Like V Domains and Bispecific Molecules for Trispecific Trimer Constructs | | |
|---|---|---|
| | | SEQ ID NO. |
| #4470 | 5' GCT GGA TTG TTA TTA CTC GCG GCC CAG CCG GCC ATG GCC GCA ATG CAC GTG GCC CAG CCT GCT GTG | 20 |
| #4471 | 5' GAA ATA AGC TTG AAT CTT CAG AGG AGC GGT TCC GTT GCC TAT GCC CAG GTA | 21 |
| #4472 | 5' TAC CTG GGC ATA GGC AAC GGA ACC GCT CCT CTG AAG ATT CAA GCT TAT TTC | 22 |
| #4473 | 5' CCT GGG GAT GAG TTT TTG TTC TGC GGC CGC TTC AGG TTG ACT GAA GTT AGC AAG | 23 |
| #4474 | 5' GCT GGA TTG TTA TTA CTC GCG GCC CAG CCG GCC ATG GCC CAG GTG AAG CTG GTG GAG TCT GGG | 24 |
| #4475 | 5' GAA ATA AGC TIG AAT CTT CAG AGG AGC TGC AGA GAC AGT GAC CAG AGT CCC | 25 |
| #4477 | 5' CCT GGG GAT GAG TTT TTG TTC TGC GGC CGC TTC AGG TTG ACT GAA GTT AGC AAG | 26 |
| #4480 | 5' TAC CTG GGC ATA GGC AAC GGA ACC GAT ATC CAG ATG ACA CAG TCT CCA | 27 |
| #4481 | 5' CCT GGG GAT GAG TTT TTG TTC TGC GGC CGC CCG TTT TAT TTC CAA CTT TGT CCC | 28 |

Human CTLA-4 and CD86 (Aruffo and Seed 1987) were joined into a 0-linker gene construct by a linking PCR with oligonucleotides #4470 & #4473. Human CTLA-4 and UV-3 VL were joined into 0-linker gene construct by a linking PCR with oligonucleotides #4478 & # 4471 and UV-3 VH and human CD86 were joined into 0-linker gene construct by a linking PCR with oligonucleotides #4474 & #4477. This produced ligation between codons for C-terminal UV-3 V_{H}-Ala¹¹⁴ and N-terminal CD86-Ala¹ by PCR overlap-extension. The Ig-like V domain 0-linker gene constructs were cloned into the *Sfi*1-*Not*1 sites of the expression vector pGC, which provides an N-terminal pelB leader sequence and C-terminal FLAG octapeptide tag tail (Coia *et al,* 1996). Ligation between codons for C-terminal CTLA-4-Ala¹¹² and N-terminal CD86-Ala1 by PCR overlap-extension produced Ig-like V domain 0-linker gene constructs which were cloned into the *Sfi*1-*Not*1 sites of the expression vector pGC. Ligation between codons for C-terminal CTLA-4-Ala¹¹² and N-terminal UV-3-VL-Glu¹ by PCR overlap-extension was used to produce the Ig-like V domain 0-linker gene construct, which was cloned into the *Sfi*1-*Not*1 sites of the expression vector pGC. The C-terminus of the V_{L} was immediately followed by the FLAG^{™} sequence for affinity purification.

The entire DNA sequence of the cloned Ig-like V domains with 0-linkers was determined, using DNA purified by alkaline lysis and sequencing reactions performed using the PRISM Cycle Sequencing Kit (ABI). This confirmed that the Ig-like V domain 0-linker gene constructs comprised direct ligation between codons for each of the domains. Expression was as described in Example 5. Gel filtration of affinity-purified CTLA-4-0-CD86, CTLA-4-0-UV-3 VL or UV-3 VH-0-CD86 by FPLC on a calibrated Superose 12 column revealed major peaks at -20.00 mins for each construct (data not shown),consistent with the retention time of trimer. 8M urea or other disaggregating reagents are used to dissociate and prevent the formation of homotrimers. Mixing the purified CTLA-4-0-CD86, CTLA-4-0-UV-3 VL and UV-3 VH-0-CD86 Ig-like V domains and removing the disaggregating reagent by gel filtration or dialysis forms the trispecific trimer.

### DISCUSSION

### Design of scFv-0 molecules lacking a foreign flexible linker polypeptide

The design of V_{H}-V_{L} and V_{L}-V_{H} ligations was initially based on the precise distances between N- and C-terminal residues from the crystal structure of NC10 scFv-15 (Kortt *et al,* 1994). Previous studies have investigated the design of flexible linker peptides to join V_{H} and V_{L} domains to produce scFvs (Huston *et al,* 1991; Ragg and Whitlow, 1995), and the effect of the linker structure on the solution properties of scFvs (Holliger *et al,* 1993; Desplancq *et al,* 1994; Whitlow *et al,* 1994; Alfthan et *al,* 1995; Solar and Gershoni, 1995). ScFvs with the classical 15-residue linker, (Gly₄ Ser)₃ described by Huston *et al,* (1989, 1991) can exist as a monomers, dimers and higher molecular mass multimers (Holliger *et al,* 1993; Whitlow *et al,* 1994; Kortt *et al,* 1994). This propensity of scFvs to dimerise was exploited further by Whitlow *et al,* (1994) to make bispecific dimers by linking V_{H} and V_{L} domains of two different antibodies (4-4-20 and CC49) to form a mixed scFv and then forming an active heterodimer by refolding a mixture of the two scFv in the presence of 20% ethanol, 0.5 M guanidine hydrochloride. The main disadvantage of this approach with 15 residue or longer linkers is that different V_{H} and V_{L} pairings show different dimerization and dissociation rates. A variety of scFv-type constructs is illustrated in Figure 21. Four types are identified:
A: An scFv comprising V_{H}-L-V_{L} where L is a linker polypeptide as described by Whitlow et al and WO 93/31789; by Ladner et al, US-4,946,778 and WO 88/06630; and by McCafferty *et al* and by McCartney *et al.*
B: A single polypeptide V_{H}-L1-V_{L}-L2-V_{H}-L3-V_{L} which forms two scFv modules joined by linker polypeptide L2, and in which the V_{H} and V_{L} domains of each scFv module are joined by polypeptides L1 and L3 respectively. The design is described by Chang, AU-640863 and by George et *al.*
C: Two scFv molecules each comprising V_{H}-L1-V_{L}-L2(a,b), in which the V_{H} and V_{L} domains are joined by linker polypeptide L1 and the two scFv domains are joined together by a C-terminal adhesive linkers L2a and L2b. The design is described by Pack *et al,* PI-93-258685.
D: This design of PCT/AU93/00491, which is clearly different to A, B and C above. A single scFv molecule V_{H}-L-V_{L} comprises a shortened linker polypeptide L which specifically prevents formation of scFvs of the type A, B or C, and instead forces self-association of two scFvs into a bivalent scFv dimer with two antigen combining sites (target-binding regions; TBR-A). The association of two different scFv molecules will form a bispecific diabody (TBRs-A,B).

Linkers of less than 12 residues are too short to permit pairing between V_{H} and V_{L} domains on the same chain, and have been used to force an intermolecular pairing of domains into dimers, termed diabodies (Holliger *et al,* 1993, 1996; Zhu *et al,* 1996; PCT/AU93/00491; WO 94/13804; WO 95/08577). Holliger *et al,* 1993, 1996, WO 94/13804 and WO 95/08577 described a model of scFv diabodies with V_{H} domains joined back-to-back, and suggested that these structures required a linker of at least one or two residues. This model was confirmed in a crystal structure of a 5-residue diabody (Perisic *et al,* 1994), but it was noted that scFv-0 could not be fitted to this conformation, even with severe rotations of the V_{H} domains. Desplancq *et al,* (1994) described a series of scFvs with linkers of 10, 5 and zero residues, and concluded on the basis of FPLC analyses that these scFvs were predominantly dimers with minor amounts of monomer. Alfthan *et al* (1995) also reported that scFvs with small linkers, down to 2 residues in length, formed dimers. McGuinness *et al* (1996) claimed that bispecific scFv-0 molecules were diabodies and could be displayed and selected from bacteriophage libraries. However, none of these studies performed precise molecular size determination on the expressed soluble products to confirm whether dimers were actually formed.

### scFv trimers

We have now discovered that the NC10 scFv-0 yielded a molecular mass on FPLC and sedimentation equilibrium analysis of 70 kDa, significantly higher than expected for a dimer (52 kDa), and less than that for a trimer (78.5 kDa) (Table 2). Binding experiments with anti-idiotype 3-2G12 Fab' showed that the scFv-0 formed a complex of Mᵣ of 212 kDa, consistent with three Fab' fragments binding per scFv-0. This result confirmed that the 70 kDa NC10 scFv-0 was a trimer, and that three pairs of V_{H} and V_{L} domains interact to form three active antigen-combining sites (TBRs). This scFv-0 structure showed no propensity to form higher molecular mass multimers. The NC10 scFv-0 trimer also bound to neuraminidase, but the arrangement of the antigen combining sites is such that a second antigen binding site on NC10 scFv-0 could not cross-link the neuraminidase tetramers into 'sandwiches', as shown for the scFv-10 and scFv-5 dimers in Figure 8. 11-1G10 ScFv-0 also exclusively formed trimers, which were shown to be trivalent for Fab binding by complex formation in solution (Table 4). NC10 scFv-0 (V_{L}-V_{H}) also formed trimers (Figure 17).

A computer graphic model, shown in Figure 2, was constructed for a zero residue-linked scFv trimer, based on the NC10 scFv coordinates, using circular 3-fold symmetry with the 'O' molecular graphics package (Jones *et al,* 1991), from the coordinates of the NC10 Fv domain in Protein Database entry 1NMB (Malby *et al,* 1994) and MOLSCRIPT (Kraulis, 1991). Ser 112, the C-terminal residues of V_{H} domains, were joined by single peptide bonds to Asp 1, the N-terminal residues of V_{L} domains. The V_{H} and V_{L} domains were rotated around the peptide bond to minimise steric clashes between domains. The Fv conformation and CDR positions were consistent with the molecule possessing trivalent affinity. The low contact area between Fv modules, across the V_{H}-V_{L} interface, may account for the slightly increased proteolytic susceptibility of NC10 scFv-0 trimers compared to NC10 scFv-5 dimers. Although the protein chemical data could not differentiate between symmetric or non-symmetric trimers, the model clearly demonstrated that zero-linked scFvs could form trimers without interdomain steric constraints.

In these models of NC10 scFv-0 trimers (Figures 2 and 8), and in EM images (Figure 16), the TBRs to the three Fab' molecules appear not to be planar, but are pointing towards one direction as in the legs on a tripod. Obviously, several configurations can be modelled, guided by steric constraints which limit both the flexibility of Fv modules and the proximity of three binding antigens.

In contrast, dimeric structures have been proposed for scFv-0 in which only V_{H} domains are in contact between Fv modules (Perisic *et al,* 1994). These dimeric structures impose severe steric constraints when the linker is less than 3 residues in length. Our data show that trimers are exclusively favoured over dimers for both NC10 scFv-0 and 11-1G10 scFv-0. Steric constraints probably prevent the dimer formation and result in the trimeric configuration as the generally preferred conformation for scFv-0 molecules.

### Binding affinities of scFvs

Binding studies using the BIAcore™ biosensor showed that all the scFvs tested bound to immobilised anti-idiotype 3-2G12 Fab'. In the case where the dimers and trimer were used as analyte, the kinetic constants were not evaluated because multivalent binding resulted in an avidity effect and invalidated the kinetic interaction model. Experiments with the immobilised NC10 scFv-0 showed that the affinity of each antigen combining site (TBR) for anti-idiotype 3-2G12 Fab' was essentially identical (Table 4), and that the increases in affinity shown in Figure 10 are clearly due to an avidity effect. The complex formation data in solution supported the conclusion that the scFvs bound stoichiometrically to antigen.

The gain in affinity through multivalent binding (avidity) makes these multimeric scFvs attractive as therapeutic and diagnostic reagents. Furthermore, the construction of tricistronic expression vectors enables the production of trispecific scFv-0 reagents.

In conclusion, this specification shows that linkers of 10 or 5 residues joining the NC10 V_{H} and V_{L} domains result in the exclusive formation of bivalent dimers. The pairing of V_{H} and V_{L} domains from different molecules results in non-covalently crossed diabodies. For the scFv-5 and scFv-10 constructs monomers do not form, and any observed monomeric species are proteolytically-produced Fv fragments. The direct linkage of NC10 V_{H} and V_{L} domains as scFv-0 produced a trimer, with three antigen combining sites (TBRs) capable of binding antigen. Previous scFv-0 constructs have been reported to be dimers, which suggests that C-terminus and N-terminus residues in those constructs have some flexibility and may act as a short linker (Holliger *et al*, 1993). Indeed, the allowed flexibility between Fv modules of a 5-residue linked diabody has recently been modelled (Holliger *et al*, 1996), and presumably linkers of less than 5 residues would severely restrict this flexibility.

We initially thought that the trimeric conformation was unique to NC10 scFv-0, perhaps due to steric clashes between V-domains which prevented the dimeric association. However, we show in this specification that NC10 scFv molecules linked with up to 2 flexible residues between the V-domains also form trimers. We also show that the reverse orientation, for NC10 scFv-0 V_{L}-V_{H} is a trimer, but can also be a tetramer. Furthermore, we show that a second scFv-0 in V_{H}-V_{L} orientation, constructed from the anti-idiotype 11-1G10 antibody, can be a trimer, and possess trivalent specificity. We also show that a third scFv-0 in V_{H}-V_{L} orientation, constructed from the C215 antibody, can also form a trimer.

This specification describes methods of producing trimeric scFv-0 molecules constructed by direct ligation of two immunoglobulin-like domains, including but not limited to scFv-0 molecules in V_{H}-V_{L} and V_{L}-V_{H} orientations, and teaches the design of polyspecific reagents.

Ig-like V domains of non-antibody origin have also been joined without a linker in a construct equivalent to the scFv-0 to form trimers, and we have shown here the joining of CD86 (Ig-like V domain) to CTLA-4 (Ig-like V domain), as well as joining each of these to UV-3 VH and UV-3 VL respectively. The trimer formation by each of these constructs teaches that polyspecific and in this case trispecifc trimers can form as shown in Figure 1 Aspect II, with the VH and VL of UV-3 noncovalently associating, the two CD86 Ig-like V domains noncovalently associating, and the two CTLA-4 Ig-like domains noncovalently associating.

### Design of polyvalent reagents

In the design of the trimeric NC10 scFv-0 residues Ser¹¹² and Asp¹ were ligated as a direct fusion of domains and, presumably, the absence of a flexible linker prevents the dimeric configuration. The C-terminal residue Ser¹¹² was chosen from precise structural data, obtained by crystallographic analysis (Malby *et al,* 1994), as being immediately adjacent to the last residue constrained by hydrogen bonding to the V_{H} domain framework before the start of the flexible hinge region. Similarly, Asp¹ of V_{L} was known to be hydrogen-bonded to the V-domain framework and was close to the antigen-binding site, but was not involved in antigen binding. Using a similar rationale, the NC10 scFv-0 V_{L}-V_{H} molecules were synthesised as a direct ligation of the C-terminal V_{L} residue Arg¹⁰⁷ to the N-terminal V_{H} residue Gln¹ (residues taken from Malby *et al,* 1994), and shown to associate into a stable trimer by FPLC analysis (Figure 17).

Since there are no structural data for 11-1G10, we assumed from structural homology that direct ligation of V_{H}-Ser¹¹³ to V_{L}-Gln¹ would similarly prevent the formation of a flexible linker, unless there is unfolding of the terminal β-stands from the V-domain framework. The 11-1G10 scFv-0 exclusively formed trimers (Figure 13), which were shown to be fully active and trivalent for Fab binding by complex formation in solution (Figure 14). In contrast, the 11-1G10 scFv-15 preferentially formed monomers with a small percentage of dimers, consistent with most previous observations of scFv-15 structures. The slight difference between calculated and experimental molecular masses determined by gel filtration and sedimentation equilibrium is within the usual error range for these analytical methods (Table 5). As expected, binding experiments with the immobilised NC41 Fab on the BIAcore biosensor showed that the trimer had a slower dissociation rate compared to the monomer, which can be attributed to the increased avidity of multivalent binding (Figure 15).

Taken together, our examples of scFv-0 molecules demonstrate that directly ligated V_{H}-V_{L} or V_{L}-V_{H} domains form trimeric scFv-0 molecules and in some cases, form a tetramer. The residues chosen for ligation of V_{H}-V_{L} or V_{L}-V_{H} should be close to the V-domain framework, and can either be determined experimentally, or can be predicted by homology to known Fv structures (Malby *et al*, 1994). Presumably, additional residues that form a more flexible linker will allow the formation of diabodies (Holliger *et al,* 1993; PCT/AU93/00491; WO 94/13804; WO 95/08577).

ScFv-0 molecules can be easily modelled into a symmetric trimeric conformation without interdomain steric constraints (Figure 2). In this model of NC10 scFv-0, the Fab arms of the trimer/Fab complex are not extended in planar configuration, but are angled together in one direction and appear as the legs of a tripod. Obviously, alternative configurations can be modelled, guided by steric constraints which limit both the flexibility of Fv modules and the proximity of three binding antigens. Unfortunately, protein chemical data alone cannot differentiate between symmetrical or non-symmetrical trimer configurations.

It will be appreciated by those skilled in the art that the effect of V-domain orientation and the requirement up to two residues in the flexible linker may be different for other scFv molecules, but that the preferred linker length and V-domain orientation can be easily determined using the designed iterative alterations described in this specification.

### Applications

This specification predicts that the polymeric configuration, and particularly trimers and tetramers, will be the preferred stable conformation in many other scFv-0 molecules. The increased tumour to blood ratio reported for bivalent scFv dimers over monomers (Wu *et al,* 1996), presumably resulting from higher avidity and reduced clearance rates, offers advantages for imaging, diagnosis and therapy. The further gain in affinity through avidity makes trimeric and tetrameric scFvs attractive for *in vivo* imaging and tumour penetration as an alternative reagent to diabodies (Wu *et al,* 1996) and multivalent chemical conjugates (Antoniuw *et al,* 1996, Casey et *al,* 1996; Adams *et al,* 1993; McCartney et al, 1995).

The design of bivalent diabodies directly led to the design of bispecific diabodies using dicistronic vectors to express two different scFv molecules *in situ,* V_{H}A-linker-V_{L}B and V_{H}B-linker-V_{L}A, which associate to form TBRs with the specificities of the parent antibodies A and B from which the V-genes were isolated (Holliger *et al,* 1993, 1996; WO 94/13804; WO 95/08577). The linker sequence chosen for these bispecific diabodies, Gly₄Ser, provided a flexible and hydrophilic hinge.

In a similar process, and using the inventive steps described in this specification, tricistronic vectors can be designed to express three different scFv-0 molecules *in situ,* V_{H}A-V_{L}B, V_{H}B-V_{L}C, and V_{H}C-V_{L}A which will associate to form a trispecific trimer with TBRs equivalent to the parent antibodies A,B,C from which the V-genes have been obtained. The three V_{H}-V_{L} ScFv-0 molecules can associate into a trispecific trimer in a schematic configuration similar to that shown in Figure 2. It will be readily appreciated that purification of the trispecific molecules to homogeneity is likely to require three sequential affinity columns to select either for three active TBRs or to select for individual epitope-tagged molecules. It will also be appreciated that the reverse orientation V_{L}-V_{H} is a suitable alternative configuration. The construction of tricistronic expression vectors will enable the production of trispecific scFv-0 reagents with applications including, but not limited to T-cell recruitment and activation.

Similarly, tetramers with four active TBRs can be formed by association of four scFv identical molecules, and tetraspecific tetrabodies can be formed by association of four different scFv molecules, preferably expressed simultaneously from tetracistronic vectors.

It will be apparent to the person skilled in the art that while the invention has been described in some detail for the purposes of clarity and understanding, various modifications and alterations to the embodiments and methods described herein may be made without departing from the scope of the inventive concept disclosed in this specification.

Reference cited herein are listed on the following pages, and are incorporated herein by this reference.

### REFERENCES

Adams, G.P., McCartney, J.E., Tai, M-S., Opperman, H., Huston, J.S., Stafford, W.F., Bookman, M.A., Fand, I., Houston, L.L. and Weiner, L.M. Cancer Res., 1993 53 4026-4034.

Alfthan, K., Takkinen, K., Sizman, D., Soderlund, H. and Terri, T.T. Protein Engng., 1995 8 725-731.

Anand, N.N., Mandal, S., MacKenzie, C.R., Sadoska, J., Sigurskjold B., Young, N.M., Bundle, D. R.and Narang, S.A. J. Biol. Chem., 1991 266 21874-21879.

Antoniw P., Farnsworth A.P., Turner A., Haines A.M., Mountain, A., Mackintosh J., Shochat D., Humm J., Welt, S., Old L.J., Yarranton G.T. and King D.J. British Journal of Cancer, 1996 74 513-524

Aruffo, A. and Seed, B. Proc. Natl. Acad. Sci, USA, 1987 84 8573-8577

Atwell, J.L., Pearce, L.A., Lah, M., Gruen, L.C., Kortt, A.A. and Hudson, P. J. Molec. Immunol., 1996 33 1301 -1312

Batra, J.K., Kasprzyk, P.G., Bird, R.E., Pastan, I. and King, C.R. Proc. Natl. Acad. Sci, USA, 1992 89 5867-5871.

Bedzyk, W.D., Weidner, K.M., Denzin, L.K., Johnson, L.S., Hardman, K.D, Pantoliano, M.W., Asel, E.D. and Voss, Jr. E.W. J. Biol Chem., 1990 265 18165-18620.

Bird, R.E., Hardman, K.D., Jacobson, J.W., Johnson, S., Kaufman, B.M., Lee, S.-M., Lee, T., Pope, H.S., Riordan, G.S. and Whitlow, M. Science, 1988 242 423-426.

Brizzard, B.L., Chubte, R.G. and Vizard, D.L. BioTechniques., 1994 16 730-734.

Buchner, J., Pastan, I. and Brinkman, U. Anal. Biochem., 1992 205 263-270.

Casey, J.L., King, D.J., Chaplin, L.C., Haines, A.M., Pedley, R.B., Mountain, A., Yarranton, G.T. and Begent, R.H. British Journal of Cancer, 1996, 74 1397-1405

Chaudhary, V.K., Queen, C., Jungans, R.P., Waldmann, T.A., Fitzgerald, D.J. and Pastan, I. Nature (London), 1989 339 394-397.

Chaudhary, V.K., Batra, J.K., Gallo, M.G., Willingham, M.C., Fitzgerald, D.G. and Pastan, I. Proc. Natl. Acad. Sci. USA, 1990 87 1066-1070.

Clackson et al J. Mol. Biol., 1991 352 624-28

Coia, G., Hudson, P.J. and Lilley, G.G. J. Immunol. Meth., 1996 192 13-23.

Colman, P.M. (1989) in The influenza viruses (Krug, R.M., ed) pp. 175-218, Plenum Press, New York and London.

Colman, P.M., Tulip, W.R., Varghese, J.N., Baker, A.T., Tulloch, P.A., Air, G.M. and Webster, R.G. Phil. Trans. Roy. Soc. Lond. ser B., 1987 323 511-518.

Cumber, A.J., Ward, E.S., Winter, G., Parnell, G. and Wawrzynczak, E.J. J. Immunol., 1992 149 120-126.

Dariavach, P., Mattei, M.G., Golstein, P., and Lefranc, M. P. Eur.J.Immunol. 1988 18 1901-1905.

Desplancq, D., King, D.J., Lawson, A.D.G. and Mountain, A. Protein Engng., 1994 7 1027-1033.

Dubel, S., Breitling, F., Kontermann, R., Schmidt, T., Skerra, A. and Little, M. J. Immunol. Methods, 1995 178 201-209.

Ducancel, F., Gillet, D., Carrier, A., Lajeunesse, E., Menez, A. and Boulain, J.C. Bio/technology, 1993 11 601-605.

Figini, M., Marks, J. D., Winter, G. and Griffiths, A. D. J. Mol .Biol. 1994 239 68-78

Forsberg, G., Fosgren, M., Jaki, M., Norin, M., Sterky,C., Enhorning, A., Larsson,K., Ericsson,M. and Bjork,P. J. Biol. Chem., 1997 272 12430-12436

Glockshuber, R., Malia, M., Pfitzinger, I. and Pluckthun, A. Biochemistry, 1990 29 1362-1367.

Gruber, M., Schodin, B.A., Wilson, E.R. and Kranz, D.M. J. Immunol., 1994 152 5368-5374.

Gruen, L.C., Kortt, A.A. and Nice, E. Eur. J. Biochem., 1993 217 319-325.

Holliger, P., Prospero, T. and Winter, G. Proc. Natl. Acad..Sci. USA, 1993 90 6444-6448.

Holliger, P., Brissinick, J., Williams, R.L., Thielemans, K. and Winter, G. Protein Engng., 1996 9 299-305.

Hoogenboom, H., Griffiths, A., Johnson, K., Chiswell, D., Hudson, P. and Winter, G. Nucleic Acids Res., 1991 19 4133-4137

Hoogenboom et al Nucl. Acids. Res., 1996 19 4133-4137

Hopp, T.P., Prickett, K.S., Libby, R.T., March, C.J., Cerretti, D.P., Uradl, D.L. and Conlon, P.J. Bio/Technology, 1988 6 1204-1210.

Hudson, P.J., Malby, R., Lah, M., Dolezal, O., Kortt, A.A., Irving, R.A., and Colman P.M. J. Cell Biochem. Supp., 1994 18D 206.

Hudson, P. J., (1995) in Monoclonal Antibodies: The Second Generation. BIOS Scientific Publications Oxford U.K. (ed H. Zola) pp. 183-202

Huston, J.S., Levison, D., Mudgett-Hunter, M., Tai, M.-S., Novotny, J., Margolies, M.N., Ridge, R.J., Bruccoleri, R.E., Haber, E., Crea, R. and Oppermann, H. Proc. Natl. Acad. Sci. USA, 1988 85 5879-5883.

Huston, J.S., Mudgett-Hunter, M., Tai,M.-S., McCartney, J.E., Warren, F.D., Haber, E., and Oppermann, H. Methods Enzymol., 1991 203 46-88

Irving, R.A., Kortt A.A. and Hudson, P.J. Immunotechnology, 1996 2 127-143

Jones, T.A., Zou, J-Y., Cowan, S.W. and Kjeldgaard, M. Acta Cryst., 1991 A47 10-119.

Jonsson, U., Fagerstam, L., Lofas, S., Stenberg, E., Karlsson, R., Frostel, A., Markey, F. and Schindler, F. Ann. Biol. Clin., 1993 51 19-26.

Kang et al Proc. Natl. Acad. Sci. USA, 1991 88 4363-466

Karlsson, R., Michelsson, A. and Mattsson, L. J. Immunol. Methods, 1991 145 229-240.

Karlsson, R., Roos, H., Fagerstam, L. and Persson, B. Methods Enzymol., 1994 6 99-100.

King, D., Byron, O.D., Mountain, A., Weir, N., Harvey, A., Lawson, A.D.G., Proudfoot, K.A., Baldock, D., Harding, S.E., Yarranton, G.T. and Owens, R.J Biochem. J., 1993 290 723-729.

Kipriyanov, S.M., Dubel, S., Breitling, F., Kontermann, R.E. and Little, M. Mol. Immunol., 1994 31 1047-1058

Kortt, A.A., Malby, R.L., Caldwell, J.B., Gruen. L.C., Ivancic, N., Lawrence, M.C., Howlett, G.J., Webster, R.G., Hudson, P.J. and Colman, P.M. Eur. J. Biochem., 1994 221 151-157.

Kraulis, P.J. Appl. Cryst. 1991 24 946-950.

Laver, W.G. Virology, 1978 86 78-87.

Lilley, G.G., Dolezal, O., Hillyard, C.J., Bernard, C. and Hudson, P.J. J. Immun. Methods, 1994 171 211-226.

Linsley, P.S., Greene, J.L., Brady, W., Bajorath, J., Ledbetter, J.A. and Peach, R. Immunity, 1994 1 793-801.

Linsley, P.S. Ledbetter, J., Peach, R., Bajorath, J. Immunology, 1995 146 130-140.

McCafferty et al Nature 1991 348 552

McCartney, J.E., Tai, M-S., Hudziak, R.M., Dams, G.P., Weiner, L.M., Jin, D., Stafford, W.F., Liu, S., Bookman, M.A., Laminet, A.A., Fand, I., Houston, L.L., Oppermann, H. and Huston, J.S. Protein Engng., 1995 8 310-314.

McGregor, D.P., Molloy, P.E., Cunningham, C. and Harris, W.J. Mol. Immnuol., 1994 31 219-26.

McGuinness, B.T., Walter, G., FitzGerald, K., Schuler, P., Mahoney, W., Duncan, A.R. and Hoogenboom, H.R. Nature Biotechnology, 1996 14 1149-1154

McKimm-Breschkin, J.L., Caldwell, J. B., Guthrie, R.E. and Kortt, A.A. J. Virol. Methods, 1991 32 121-124.

Mack, M., Riethmuller, G. and Kufer, P. Proc. Natl. Acad. Sci. USA, 1995 92 7021-7025.

Malby, R.L., Caldwell, J.B., Gruen, L.C., Harley, V.R., Ivancic, N., Kortt, A.A., Lilley, G.G., Power, B.E., Webster, R.G., and Colman, P.M., Hudson, P.J. Proteins: Struct. Func. Genet., 1993 16 57-63.

Mallender, W.D. and Voss, Jr. E.W. J.Biol. Chem., 1994 269 199-206.

Marks et al J. Mol. Biol., 1991 222 581-597

Marks et al Bio/Technology, 1992 10 779-783

Metzger, D.W. and Webster, R.G. (1990) in Idiotype Networks in Biology and Medicine (Osterhaus, A.D.M.E. and Uytdellaag, F.G.C.M. ed) pp. 257-267, Elsevier Publishers B.V. (Biomedical Division)

Neri, D., Momo, M., Prospero, T. and Winter, G.. J. Mol. Biol., 1995 246 367-373.

Pack, P., Muller, K., Zahn, R. and Pluckthun, A. J. Mol. Biol., 1995 246 28-34

Pantoliano, M.W., Bird, R.E., Johnson., S., Asel, E.D., Dodd, S.W., Wood, J.F. and Hardman, K.D. Biochemistry, 1991 30 10117-10125.

Pack, P and Pluckthun, A. Biochemistry, 1992 31 1570-1584.

Pack, P., Kujau, M., Schroeckh, V., Knupfer, U., Wenderoth, R., Rusenberg, D. and Pluckthun, A. Bio/technology, 1993 11 1271-1277.

Perisic, O., Webb, P.A., Holliger, P., Winter, G. and Williams, R.L. Structure, 1994 2 1217-1226.

Ragg, R. And Whitlow, M. FASEB J., 1995 9 73-80

Schott, M.E., Fruzier, K.A., Pollock, D.K. and Vernaback, K.M. Bioconjugate Chem., 1993 4 153-165

Takkinen K., Laukanen, M.-L., Sizmann D., Alfthan, K., Immonen, T., Vanne, L., Kaartinen., J.K.C. and Teeri, T.T. Protein Engng., 1991 7 837-841.

Tulip, W.R., Varghese, J.N., Laver, W.G., Webster, R.G. and Colman, P.M. J. Mol. Biol., 1992 227 122-148.

Tulloch, P.A., Colman, P.M., Davis, P.C., Laver, W.G., Webster, R.G. and Air, G.M. J. Mol. Biol., 1986 190 215-225.

Van Holde, K. E. (1975) in The proteins, (Neurath, H. and Hill, R. L. eds) vol 1, pp. 225-291, Academic Press, New York.

Ward, C.W., Murray, J.M., Roxburgh, C.M. and Jackson, D.C. Virology, 1983 126 370-375.

Wels, W., Harweth, I.-M., Zwickl, M., Hardman, N., Groner, B. and Hynes, N.E. Bio/Technology, 1992 10 1128-1132.

Whitlow, M., and Filpula, D. Methods: A Companion to Methods Enzymol., 1991 2 97-105. Whitlow, M., Bell, B.A., Feng, S.-L., Filpula, D., Hardman, K.D., Hubert, S.L., Rollence, M., Wood, J.F., Schott, M.E., Milencic, D.E., Yokota, T. and Scholm, J. Protein Eng., 1993 6 989-995.

Whitlow, M., Filpula, D., Rollence, M.L., Feng, S.-L. Woods, J.F. Protein Engng., 1994 7 1017-1026.

Wu, A.M., Chen, W., Raubitschek, A., Williams, L.E., Neumaier, M., Fischer, R., Hu, S-Z., Odom-Maryon, T., Wong, J.Y.C and Shively, J.E. Immunotechnology., 1986 2 21-36.

Zdanov, A., Li, Y., Bundle, D.R., Deng, S-J., MacKenzie, C.R., Narang, S.A., Young, N.M. and Cygler, M. Proc. Natl. Acad. Sci. USA, 1994 91 6423-6427.

Zhu, Z., Zapata, G., Shalaby, R, Snedecor, B., Chen, H. and Carter, P. Nature Biotechnology, 1996 14 192-196

The present invention also provides:
1. A polyvalent or polyspecific protein complex of the invention, comprising a non-immunoglobulin-like domain.
2. A polyvalent or polyspecific protein complex of the invention, in which the TBRs of each of the monomer polypeptides are respectively directed to three separate targets, whereby the complex possesses a plurality of separate specificities.
3. A polyvalent or polyspecific protein complex of the invention, comprising identical polypeptides, each of which comprises immunoglobulin V_{H} and V_{L} domains which are covalently joined preferably without a polypeptide linker, in which the polypeptides associate to form active TBRs specific for the same target molecule.
4. A polyvalent or polyspecific protein complex according to embodiment 3, comprising identical scFv molecules which are inactive as monomers, but which form active and identical antigen combining sites in the complex.
5. A polyvalent or polyspecific protein complex according to embodiment 2, comprising different scFv molecules which are inactive as monomers, but which form active and different antigen combining sites in the complex.
6. An imaging reagent comprising a polyvalent or polyspecific protein of the invention.
7. An imaging reagent according to embodiment 6, in which all the TBRs of the polyvalent or polyspecific protein are directed to a molecular marker specific for a pathological condition, and in which the protein is either labelled with radioisotopes or is conjugated to a suitable imaging reagent.
8. An imaging reagent according to embodiment 6, in which two TBRs of the polyvalent or polyspecific protein are directed to two different markers specific for a pathological condition or site, and a third is directed to a suitable imaging reagent.
9. An imaging reagent according to embodiment 6, in which one TBR of the polyvalent or polyspecific protein is directed to a marker characteristic of a pathological condition, a second TBR is directed to a marker specific for a tissue site where the pathological condition is suspected to exist, and a third TBR is directed to a suitable imaging agent.
10. An imaging reagent according to embodiment 6, in which one TBR of the protein is directed to a marker characteristic of the pathological condition and the remaining TBRs are directed to different imaging agents.
11. An imaging reagent according to any one of embodiments 6 to 10, in which the polyvalent or polyspecific protein is a trimer or a tetramer.
12. An imaging reagent according to any one of embodiments 6 to 10, in which the molecular marker is specific for a tumour.

## Claims

1. A polyvalent or polyspecific protein complex, comprising three or more polypeptides which associate to form three or more functional target-binding regions (TBRs), and in which each individual polypeptide comprises two or more immunoglobulin-like domains which are covalently joined together, such that two Ig-like domains in a single polypeptide do not associate with each other to form a TBR.

2. A polyvalent or polyspecific protein complex according to Claim 1 in which the immunoglobulin-like domains are linked by a peptide of fewer than 3 amino acid-residues or covalently joined without a linker peptide.

3. A polyvalent or polyspecific protein complex according to Claim 1 or Claim 2, comprising polypeptides in which each polypeptide comprises two or more immunoglobulin-like domains, and in which the domains are covalently joined without requiring a foreign linker polypeptide.

4. A polyvalent or polyspecific protein complex according to any one of Claims 1 to 3, in which the polypeptides comprise the immunoglobulin-like domains of any member of the immunoglobulin superfamily.

5. A polyvalent or polyspecific protein complex according to any one of Claims 1 to 4, in which the immunoglobulin-like domain is derived from an antibody, a T-cell receptor fragment, CD4, CD8, CD80, CD86, CD28, or CTLA4.

6. A polyvalent or polyspecific protein complex according to any one of Claims 1 to 5, comprising different polypeptides, each of which comprises antibody V_{H} and V_{L} domains or other immunoglobulin domains, which are covalently joined preferably without a polypeptide linker, and in which the polypeptides associate to form active TBRs directed against different target molecules.

7. A polyvalent or polyspecific protein complex according to any one of Claims 1 to 5, comprising two polypeptides which may be the same or different, each polypeptide comprising two or more immunoglobulin-like domains, in which the polypeptides associate to form a trimer with three or more active TBRs directed against different molecules.

8. A polyvalent or polyspecific protein complex according to any one of Claims 1 to 7, in which the TBRs of each of the monomer polypeptides are respectively directed to three separate targets, whereby the complex possesses a plurality of separate specifities.

9. A polyvalent or polyspecific protein complex according to any one of Claims 1 to 5, comprising identical polypeptides, each of which comprises immunoglobulin V_{H} and V_{L} domains which are covalently joined preferably without a polypeptide linker, in which the polypeptides associate to form active TBRs specific for the same target molecule.

10. A polyvalent or polyspecific protein complex according to any one of Claims 1 to 9, which is a trimer or a tetramer.

11. A recombinant polyvalent protein complex comprising identical single chain variable fragment (scFv) polypeptides, each of which comprises immunoglobulin V_{H} and V_{L} domains which associate to form a tetramer with four identical target-binding regions (TBRs) specific for the same target molecule, and in which each individual polypeptide is covalently joined together, such that the V_{H} and V_{L} domains in a single polypeptide do not associate with each other to form a TBR, wherein the V_{H} and V_{L} domains are linked by a peptide of one or two amino acid residues.

12. A recombinant polyvalent protein complex comprising identical single chain variable fragment (scFv) polypeptides, each of which comprises immunoglobulin V_{H} and V_{L} domains which associate to form a trimer or tetramer with target-binding regions (TBRs) directed against different molecules, and in which each individual polypeptide is covalently joined together, such that the V_{H} and V_{L} domains in a single polypeptide do not associate with each other to form a TBR, wherein the V_{H} and V_{L} domains are linked by a peptide of one or two amino acid residues.

13. A polyvalent or polyspecific protein complex according to any one of Claims 1 to 12, in which one or more of the polypeptides is linked to a biologically-active substance, a chemical agent, a peptide, a protein or a drug.

14. A pharmaceutical composition comprising a polyvalent or polyspecific protein complex according to any one of Claims 1 to 13, together with a pharmaceutically-acceptable carrier.

15. Use of a polyvalent or polyspecific protein complex according to any one of Claims 1 to 13 as a medicament, and/or for the manufacture of a medicament, for treating a pathological condition, wherein one or more TBRs of the protein is directed to a marker which is: a) characteristic of an organism which causes the pathological condition, or b) characteristic of a cell of the subject which manifests the pathological condition, and another TBR of the protein binds specifically to a therapeutic agent suitable for treatment of the pathological condition.

16. An imaging reagent comprising a polyvalent or polyspecific protein according to any one of Claims 1 to 13.


SEQUENCE LISTING

<110> AVIPEP PTY LIMITED
      HUDSON, Peter John
      KORTT, Alex Andrew
      IRVING, Robert Alexander
      ATWELL, John Leslie

<120> HIGH AVIDITY POLYVALENT AND POLYSPECIFIC REAGENTS

<130> P22043EP1/SJB

<150> 98910534.1
<151> 1998-03-26

<150> AU1997PO05917
<151> 1997-03-27

<160> 28

<170> PatentIn Ver. 2.1

<210> 1
<211> 68
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 1
gtcaccgtct ccggtggtgg tggttcgggt ggtggtggtt cgggtggtgg tggttcggat 60
atcgagct                                                          68


<210> 2
<211> 42
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 2
ccctggtgcc agtggcagag gccactatag ctcgagtgtg tc                    42


<210> 3
<211> 45
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 3
gggaccacgg tcaccgtctc cggtggtgat atcgagctca cacag                 45


<210> 4

<211> 45
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 4
ccctggtgcc agtggcagag gccaccacta tagctcgagt gtgtc                45


<210> 5
<211> 48
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 5
gggaccacgg tcaccgtctc cggtggtggt gatatcgagc tcacacag             48


<210> 6
<211> 48
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 6
ccctggtgcc agtggcagag gccaccacca ctatagctcg agtgtgtc             48


<210> 7
<211> 51
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 7
gggaccacgg tcaccgtctc cggtggtggt ggtgatatcg agctcacaca g         51


<210> 8
<211> 51
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 8
ccctggtgcc agtggcagag gccaccacca ccactatagc tcgagtgtgt c          51

<210> 9
<211> 23
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 9
tacatgcagc tcagcagcct gac                                         23

<210> 10
<211> 30
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 10
gatatacata tgcaggtcca actgcagcag                                  30

<210> 11
<211> 30
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 11
attaggcggg ctgaattctt atttatcatc                                  30

<210> 12
<211> 59
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 12
gcagaggcca ccaccaccaa gcccaccacc accaagccca ccaccaccaa gcctatagc  59

<210> 13
<211> 66
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 13
gctggattgt tattactcgc ggcccagccg gccatggccg caatgcacgt ggcccagcct 60
gctgtg                                                            66


<210> 14
<211> 51
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 14
gaaataagct tgaatcttca gaggagcggt tccgttgcct atgcccaggt a          51


<210> 15
<211> 51
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 15
tacctgggca taggcaacgg aaccgctcct ctgaagattc aagcttattt c          51


<210> 16
<211> 54
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 16
cctggggatg agtttttgtt ctgcggccgc ttcaggttga ctgaagttag caag        54


<210> 17
<211> 63
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 17

gctggattgt tattactcgc ggcccagccg gccatggccc aggtgaagct ggtggagtct 60
ggg 63

<210> 18
<211> 51
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 18
gaaataagct tgaatcttca gaggagctgc agagacagtg accagagtcc c 51

<210> 19
<211> 54
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 19
cctggggatg agtttttgtt ctgcggccgc ttcaggttga ctgaagttag caag 54

<210> 20
<211> 48
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 20
tacctgggca taggcaacgg aaccgatatc cagatgacac agtctcca 48

<210> 21
<211> 54
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 21
cctggggatg agtttttgtt ctgcggccgc ccgttttatt tccaactttg tccc 54

<210> 22
<211> 53
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 22
gtcaccgtct ccggtggtgg tggttcgggt ggtggtggtt cggatatcga gct          53


<210> 23
<211> 44
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 23
gcagaggcca ccaccaccaa gcccaccacc accaagccta tagc                    44


<210> 24
<211> 38
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 24
gtcaccgtct ccggtggtgg tggttcggat atcgagct                           38


<210> 25
<211> 29
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 25
gcagaggcca ccaccaccaa gcctatagc                                     29


<210> 26
<211> 23
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 26
gtcaccgtct ccgatatcga gct                                           23

<210> 27
<211> 14
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 27
gcagaggcta tagc                                                    14


<210> 28
<211> 42
<212> DNA
<213> Artificial Sequence

<220>
<223> Description of Artificial Sequence:synthetic
      oligonucleotide duplex encoding peptide linker
      inserted into the BstEII adn SacI restriction
      sites of pPOW-scFv NC10

<400> 28
gggaccacgg tcaccgtctc cggtgatatc gagctcacac ag                     42